# EUROPÄISCHE PATENTSCHRIFT

(11) **EP 2 462 894 B1**
(45) Veröffentlichungstag und Bekanntmachung des Hinweises auf die Patenterteilung: **25.10.2017**
(21) Anmeldenummer: 11401657.9
(22) Anmeldetag: 06.12.2011
(51) Int. Cl.: A61C 13/00, A61C 5/77

(54) **Verfahren zur Herstellung von dentalen Werkstücken**
Method for manufacturing dental workpieces
Procédé de fabrication de pièces dentaires

(30) Priorität: 08.12.2010 DE 102010061116
(43) Veröffentlichungstag der Anmeldung: 13.06.2012
(73) Patentinhaber: Arndt, Helge, 30989 Gehrden (DE)
(72) Erfinder: Arndt, Helge, 30989 Gehrden (DE)
(74) Vertreter: Scheffler, Jörg

(56) Entgegenhaltungen:
- EP-A1- 0 182 098
- DE-A1- 10 156 156
- DE-A1- 19 930 859
- DE-A1-102005 001 600

## Beschreibung

Die Erfindung betrifft ein Verfahren zur Materialabtragenden Bearbeitung eines insbesondere flächigen Rohlings zur Herstellung von dentalen Werkstücken, wie insbesondere von Inlays, Kronen, Brücken, Stegen, Implantatsuprakonstruktionen, Prothesen, Modellen oder dergleichen, bei dem mit einem rotierenden Werkzeug abwechselnd eine Kroneninnenseite des Werkstücks in der Intrados-Position des Rohlings und eine Kronenaußenseite in der Extrados-Position des Rohlings bearbeitet wird, wobei das rotierende Werkzeug, insbesondere ein Fräswerkzeug, Bohrer oder Schleifwerkzeug sowohl stirnseitig als auch mantelseitigmitdem Werkstück in Eingriff gebracht wird, und wobei eine gleichmäßige Nutzung der gesamten Schneidfläche angestrebt wird und damit ein mantelseitiger Eingriff bevorzugt wird.

Die Herstellung dentaler Restaurationen findet vornehmlich durch Materialabtragende Bearbeitungmitrotierenden Werkzeugenstatt. Als Rohlingwerdenhauptsächlich Rondenund Blanks verwendet. Diese haben die kleinen Rohlinge für Einzelzähne (CEREC) bis auf Glaskeramiken, Lithiumdisilikat-Feldspatkeramiken und Infiltrationskeramiken nahezuverdrängt. Poröse Zirkonoxidkeramiken, PMMA- und Composite-Kunstoffe, dentale Wachse, Kobalt-Chrom-Stähle und Titanlegierungen sind die hauptsächlich verwendeten Materialien. Diese werden mit zweischneidigen Hartmetallfräsern meist in Form vonZylindernmitHalbkugel oder kantengerundeten Zylindernbearbeitet. Mittlerweilesindvierschneidige Fräsergeplant.

Das im Lot zur Oberfläche der Ronde angeordnete Werkzeug dringt über die Z-Achs-Zustellung ins Werkstück, während über x-y-Achsvorschübe der flächige Materialabtrag stattfindet. Schrittweite wird durch erneute Z-Achs-Zustellung Schicht für Schicht abgetragen, bis das dentale Werkstück mit Haltestegen auf der einen Bearbeitungsseite (Intrados-Seite) freigelegt ist (Bearbeitung in x-y-Ebenen). Nach Wendung der Ronde wird ihre Kehrseite (Extrados-Seite)bearbeitet. Diesererste Bearbeitungsvorgang - Schruppen - wird in der Regel mit 3-mm bis 2,5-mm-Fräserndurchgeführt. Die Wiederholungdieser Bearbeitung - Schlichten -erfolgt in ein bis drei Schritten mit 2-mm-, 1,5-mm- und/oder 1-mm-Fräsern.Die größten Durchmesser der Werkzeuge bestimmen das Volumen des notwendigen zirkumferenten Freischleifens oder auch Materialverlusts.

Diese abtragende Bearbeitung entspricht, bezogen auf die Einschubachse der dentalen Restauration bzw. auf die Werkzeugachse, einer horizontalen Formung (x-y) mit schrittweise zunehmender Z-Zustellung ins Werkstück. Es bedingt, dass das Werkzeug nur mit seiner Spitze den Materialabtrag bewerkstelligt bzw. nur stirnseitig in Eingriff gebracht wird, und daraus geringere Werkzeugstandzeiten resultieren. Besonders nachteilig wirkt sich dies bei mit Diamantpartikeln bestückten Werkzeugen aus.

Einerseits steht das Werkzeug mit einer vergleichsweise kleinen Arbeitsfläche mit dem Werkstückin Kontakt, andererseitsnimmtdie Effizienzdes Abtragsder Diamantierungin Richtung Rotationsachse rapide ab. Bei der vierachsigen und fünfachsigen Bearbeitung wird diese Problematik durch Neigung des Werkzeugs vermindert.

In der Patentschrift DE 10 2005 001 600 B4 ist ein Verfahren zur materialabtragenden Bearbeitung von Werkstücken und Werkstück bzw. Formelement mit stirn- undmantelseitigen Eingriff ins Werkstück bekannt geworden, mit dem es gelingt die Seitenfläche des Werkzeugarbeitsteils zum wesentlichen Materialabtrag unter weitgehender Schonung der Werkzeugspitze zu nutzen. Diese Bearbeitung entspricht ebenfalls der horizontalen Formung, jedoch mit schrittweise abnehmender Z-Zustellung bei anfänglich maximaler Z-Zustellung ins Werkstück. Die Werkzeugspitzewirdhierbeiweitgehendergeschont. Nachteiligwirktsichdie große Anlagerungsfläche hinsichtlich der Kühlung, der elastischen Werkzeug-Verformung und der folgenden Schwingungsentstehung auf Werkstück und Werkzeug aus. Ebenso nimmt verfahrensbedingt (abnehmende Z-Zustellung) die Abnutzung der WerkzeugArbeitsfläche vom Schaft zur Werkzeugspitze kontinuierlich zu, sodass trotz erheblicher Ve r-besserung eine ungleichförmige Abnutzung stattfindet.

Das weit verbreitete und schon seit Jahrzehnten bekannte CEREC Verfahren der Firma SI-RONA entspricht einer vertikalen Formung, bei dem das Werkstück über geringe X- (bzw. Y-) Zustellung in YZ- (bzw. XZ-) Schichten schrittweise bearbeitet wird. Hierbei findet eine Z-Achsparallele das Werkstück durchdringende Abtragung von beiden Seiten (intrados und extrados) mitzwei Werkzeugengleichzeitigstatt. Alsvorteilhafterweistsichhier, dassauch die schaftnahen Arbeitsflächenanteile des Werkzeugs genutzt werden. Nachteilig wirken sich die verfahrensbedingten kleinen Werkzeugdurchmesser aus und dass die Bearbeitung parallel zur Längsachse der Restauration erfolgt, sodass insbesondere bei mehrgliedrigen Restaurationendieklassische Bearbeitungim Sinnedes Schruppensund Schlichtensaufgrund derresultierenden Instabilität der Verbindung zum Restmaterial nur unzureichend möglich ist. Ebenso führt die Bearbeitung der Kronenkavität und Kaufläche zu stärkeren Verschleiß der Werkzeugspitze. Dies resultiert aus der Abnahme der Bearbeitungseffektivität mit Kleiner werden des Quotienten aus der Z-Achs- und Y (X-)-Achs-Weglängenpro Zeitintervallundgilt gleichermaßen für die oben zitierte horizontale Formung mit abnehmender Z-Zustellung.

Des Weiteren ist zu berücksichtigen, dass bei den hier beschriebenen Verfahrendurchdie auf Werkzeugund Werkstückwirkenden Kräfte - die aus den meist senkrecht zueinander angeordneten Kraftvektorendes Vorschubsundderseitlichen Zustellungresultieren - Schwingungen bzw. Vibrationen entstehen.

Ein in der Dentalindustrie bekanntes Verfahren zur Schonung von Werkzeug und Werkstück stellt die Ultrasonic-Technologie der Fa. DMG Sauer dar. Durch die kinematische Überlagerung der Werkzeugrotation mit einer zusätzlichen Hochfrequenz-Osszillation in axialer RichtungimMikrometerbereich sollen Mikrorisse in den keramischen Werkstücken deutlich reduziertwerden, sichbessere Oberflächengütenrealisierenlassen und Werkzeugstandzeiten verlängert werden. Für die Bearbeitung von gesinterten (gehippten) Zirkon hat sich diese Technik bereits bewährt. Bei der Bearbeitung von Glas- und Lithium-Di-SilikatKeramiken werden diese Vorzüge, insbesondere im Hinblick auf die Entstehung von Mikrorissen, kontrovers diskutiert,

Üblicherweise werden die zu fräsenden Restaurationen durch Auslassung von Haltestegen anallenvier Seiten (III - VI) befestigt. Als vorteilhaft erweist sich hierbei eine statisch günstige Verankerung mitvergleichs weisewenig und kleinen Haltestegenunddie Möglichkeit, einen Rohling bis zu seinem vollständigen Verbrauch in einem Durchgang bearbeiten zu können (Schleifenim Nachtbetrieb).

Eine wirkungsvoll Werkstück sparende Bearbeitung ist mit der CAM-SoftwareHYPERDENT der Firma OPEN MIND Technologies AG bekannt geworden. Die zu fräsende Restauration wirdbeieinseitiger Werkstückbefestigung (siehe Werbung für 5-Achsmaschinen Mikron, Rödertec, Ultrasonic DMG/Sauer und Edelmetallfräsen C.HAFFNER GmbH) am äußeren Rand des Rohlings positioniert (Nesting), sodass der Materialverlust durch das notwendige zirkumferenteFreischleifen an mindestens einer Längsseite (III) meist sogar an zwei Seiten (III+IV bzw. III+V) entfällt. Die Bearbeitung erfolgt üblicherweise horizontal formend mit zunehmender Z-Zustellung. Da hierbei die Bearbeitung aller 6 Seiten (I bis VI) gleichzeitig stattfindet und nureine oderzwei Seitenals Verbindungzum Restmaterialzur Verfügungstehen, ist die Stabilität stark herabgesetzt. Um den auf das Werkstück treffenden Bearbeitungskräften - besonders im Hinblick auf Verwendung zweischneidiger Werkzeuge und auf die Hebelwirkungen bei der Bearbeitung an befestigungsfernen Stellen - entgegenzuwirken, müssendie Stegverbindungen verstärkt und vermehrt werden. EP 0 182 098 A1 und DE 199 30 859 A1 offenbaren ein Verfahren nach dem Oberbegriff von Anspruch 1. Der Erfindung liegt die Aufgabe zugrunde, insbesondere bei der Verwendung von Diamantkorn beschichteten und mehrschneidigen Werkzeugen, die Werkzeugarbeitsfläche -also die gesamte Schneidfläche - gleichmäßig zu nutzen, das heißt, die Werkzeugspitze noch weitgehender zu schonen, den mantelseitigen Eingriff auszudehnen, auf das Werkstück und Werkzeug wirkende Schwingungen und Vibrationen zu minimieren, die Werkzeugstandzeiten zu erhöhen und den Werkstückverbrauch insbesondere bei der Verarbeitung von Ronden und Blanks zu reduzieren.

Diese Aufgabe wird durch die Merkmale des Anspruchs 1 gelöst.

AlleinigeAusführungen gemäß Anspruch 1 ermöglichen den Einsatz kleinerer Werkzeugdurchmesser (beengtes Schleifen), die Minimierung des Material opfernden seitlichen Freischleifens, maximale Werkzeug-Werkstück Eingriffsflächen, gleichzeitig optimierte Kühlung und verbesserter Abtransport des Schleifstaubs, schwingungsarmes Abtragen durch parallel zum Vorschubvektoraufdas Werkzeugwirkenden Kraftvektor, Nutzunginsbes ondereder schaftnahen Anteile mit Schonung der Schneidfläche der Werkzeugspitze. Ein weiterer vorteilhafter Wirkungseffekt des Hubens ergibt sich aus der Tatsache, dass durch den Arbeitseinsatz nicht nur ein automatisches rotations symmetrisches Abrichtener folgt, sonder gleichzeitig ein parallel zur Werkzeugmantelfläche ausgerichtetes Abrichten stattfindet. Dieser vorteilhafte Wirkungseffekt beschränkt sich keineswegs nur auf mit Diamantkorn beschichtete Schleifer oder Sinterdiamanten, sondern gilt gleichermaßen für mehrschneidige Fräsen und besonders für texturgebundene Schleifer und Polierer. Letztere sowohl in weicheralsauchinharter Ausführung. Aufdiese Weisegelingtes, zum Beispiel, die Innenflächen von Teleskop-Sekundärkonstruktionen in maschinenkontrollierter Präzision auf Hochglanz zu polieren. Dazu wird die Z- (Hub-) Achse parallel zur Teleskop-Innenfläche (Funktionsfläche) ausgerichtet. Der mit Metallstift stabilisierte Hartgummipolierer umfährt hubend die teleskopierende Innenfläche der Sekundär-Krone. Hierauf erfolgt die Werkzeugvermessung in Länge und Durchmesser beispielsweise mittels laseroptischer Vermessung (Fa. BLUM). Nach entsprechender Korrektur in der Maschinen-Steuerung (Heidenhain) wird der Vorgang so lange wiederholt, bis die tolerierte Durchmesserdifferenz vorliegt.

Die Kombination des Hubens gemäß Anspruch 7 mit dem oben erwähnten horizontal formenden Verfahren mit abnehmender Z-Zustellung gemäß Patentschrift DE 10 2005001600 B4 stellt eine vorteilhafte Erweiterung dar. Hierzu würde die vorgesehene Vorschubbewegung (x-y) reduziert die Zustellung erhöht werden und die vorgesehene Z-Achseinstellung (Z=i) mit der Hubbewegung (Z=i-h) aus dem Material heraus und wieder zurück (Z=i-h+h) versehen werden. Besonders effizient ist diese Kombination, wenn bei der Bearbeitung im 4-5-Achsbetrieb die relative Werkzeug-Werkstück-Positionierung ein parallel zur Rotationsachseausgerichtetesun limitiertes Hubenerlaubt (tangentiales Bearbeiten).
Dies gilt gleichermaßen für die Bearbeitung des Kronenlumens, indem beispielsweise nach Schaffung einer Eingangskavität durch helikales Einfahren, seitliches Einschwenken und/oder Bohren bis zur maximalen Z-Zustellung zum tiefsten Punkt der Kavität mit Hub-Bearbeitung die Aufweitung der Kavität mit helikaler Vorschubbahn und abnehmender Z-Zustellung stattfindet;
Oder, dass nach Schaffung einer Eingangskavität durch helikales Einfahren geneigtes Einschwenken und/oder Bohren bis zur maximalen Z-Zustellung zum tiefsten Punkt der Kavität mit Hub-Bearbeitung die Aufweitung der zylinderförmigen Kavität zuerst über Einstellung der Neigungdes Werkzeuges parallelzurjeweiligen Kavitätenwand-Sekanten stattfindet (5-Achsbetrieb) und danach die Aufweitung unter Beibehaltung des jeweiligen Neigungswinkel mittels helikaler Vorschubbewegung stattfindet;
Oder, dass nach Schaffung einer Eingangskavität durch helikales Einfahren, seitliches Einschwenken und/oder Bohren bis zur maximalen Z-Zustellung zum tiefsten Punkt der Kavität mit ausschließlicher Hub-Bearbeitung und/oder Kombination mit dem vertikal formenden Verfahren im ersten Schritt die Aufweitung mit schlitzförmigen Abtrag über die gesamte Kavität stattfindet und anschließend die Aufweitung zu beiden Seiten nacheinander mit "S"-förmiger Vorschubbahn erfolgt.
Ebenso kann die horizontal formende Bearbeitung mit zunehmender Z-Zustellung mit dem Huben kombiniert werden (Anspruch 7). Hierzu werden die Z-Zustellungen erhöht, die Vorschübe entsprechend reduziert. Dies ist besonders für den Schrupp-Vorgang von Vorteil. Die Kombination des Hubens mit der vertikal formenden Bearbeitung kann auf verschiedenenWegenerfolgen(sieheauch Ansprüche 4 und 6). Es kann im Sinne einer verbesserten Abtragseffizienz unterstützend eingesetzt werden. Ausgehend von einer X-Zustellung und einem Z-Y-Vorschubbewegung wird letztere verlangsamt und mit einer angepassten Z-Hubbewegungüberlagert.DerHubweg variiert in Abhängigkeit der Z-Y-Koordinate und beträgt günstigerweise 20 bis 50 % der maximalen Eingriffslänge des Werkzeugs mit dem Werkstück. Die X-Zustellung wird erhöht.
Ein anderer Weg stellt das Arbeiten in größeren Zustellungs-Schichten mit stark herabgesetztem Z-Y-Vorschub dar. Die Zustellung erfolgt durch ein hubendes Eindringen in X-Richtung. Die maximale Zustellung entspricht dem Durchmesser des Werkzeugs. Anschließend erfolgt die verlangsamte seitliche Auslenkung als Z-Y-Vorschub unter ständigen Huben. Die zum Lot der Extrados- bzw. Intrados-Oberflächen geneigten Flächen der Restauration erhalten bei Zustellungen über ¼ des Werkzeugradius ein Arkadenrelief. Diese beiden Bearbeitungsverfahren gehen je nach X-Zustellung fließend ineinander über.

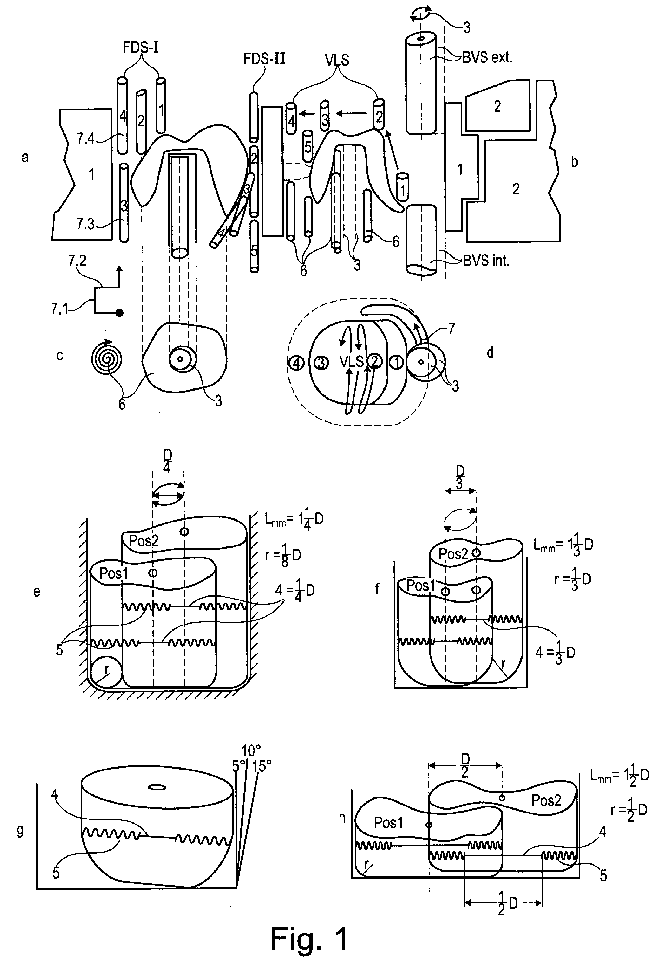

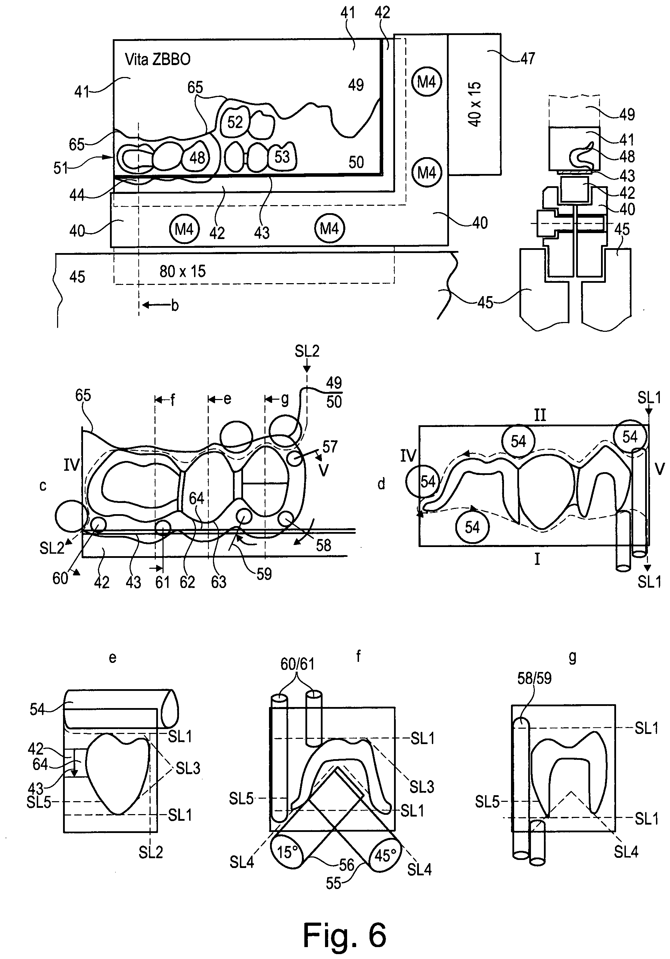

Die volle Ausschöpfung des erfindungsgemäßen Wirkungseffektes nach Anspruch 1 erhält man, indem das rotierende und hubende Werkzeug mit maximaler Z-Zustellung in der Extrados- bzw. Intrados-Position (A=0°/A=180°) mit seiner Vorschubbewegung dem Äquatorverlauf der zu schleifenden Restauration folgt (siehe Abb. 6 i und j). Darauf folgend wird diese Bearbeitung in ein oder mehreren geneigten Werkstück-Werkzeug-Positionen pro Seite wiederholt (siehe Anspruch 6). Dieses besonders für die Schrupp-Bearbeitung geeignete Vorgehen setzt eine 3+1 Achs-Austattung voraus. In Bereichen frei zugänglicher Seiten (III, evtl. IV und V) ist die komplette Schrupp-Bearbeitunggemäßdesvertikalunlimitierten Hubens möglich, insbesondere mit Verwendung größerer Werkzeugdurchmesser. In Regionendes zirkumferenten Freischleifens (der Seiten VI, evtl. IV und V, oder gar aller Seiten III-VI) findet in den geneigten Positionen ein vertikal limitiertes Huben, jedoch ohne stirnseitigen Eingriff statt. Hier sind im Sinne der Materialersparnis kleine Werkzeug-Durchmesser zu bevorzugen.
Dies ist aber auch in anderer Hinsicht relevant, da sich die Abtrags-Effizienz unabhängig vom Werkzeugdurchmesser (D) verhält. Die sich die im Eingriff befindende Schneidfläche (Aₛ) ergibtsichausder Eingriffslänge (EL) und dem Eingriffsumfang (1/2D * π); [Aₛ = 1/2D* π * EL]. Das abgetragene Materialvolumen (V_{A}) ergibt sich aus dem Produkt des Werkzeugdurchmessers (D), der Eingriffslänge (EL) und dem Vorschubweg (S) [V_{A} = D*EL*S]. Das Verhältnisdes Materialabtragszurim Eingriffbefindlichen Schneidfläche [V_{A}/Aₛ = (D*EL*S) / (1/2D* π * EL)=2S/*π] zeigtdie Unabhängigkeit des Werkzeugdurchmessers von der Abtragseffizienz auf. Dies legt nahe, in Art einer Schneidfunktion kleine Durchmesser in der Schrupp-Bearbeitungeinzusetzen. Dieser Effektwirderfindungsgemäß auch genutzt, besonders in beengten Bereichen. Bei Vorhandensein eines freien Zugangs ist dagegen den großformatigen Werkzeugen der Vorzug zugeben, sofern hier kein umfänglicher Eingriff der Mantelfläche vorliegt. Weil in diesem Fall die Schn eidflächenbelastung günstiger ausfällt.
Ein weiterer überraschender Wirkungseffekt des Hubens zeigt sich durch einen Schwingungsreduzierten Substanzabtrag, bei höheren Vorschüben, reduzierter Umlaufgeschwindigkeitundgrößeren Anlagerungsdruck. Diesistwohl einerseits auf die günstige unidirektionale Krafteinwirkungmitzusätzlicher Seitenführung, - durchdenumfänglichen Schneidflächen-Eingriffbedingt - andererseits auf den Effekt der automatischen achsparallelen Abrichtung (s.o.) und der Hub bedingten Korrekturder, durchdie Krafteinwirkungstattfindenden elastischen Auslenkungin Richtung Werkzeugendezurückzuführen. Im Gegensatzdazu findet bei der horizontal formenden Bearbeitung mit maximaler Eingriffslänge keine Nachbearbeitung des durch die elastische Verformung belassenen Materials statt, sodass das Werkzeug unter ständiger sowohl, durch den Anlagerungsdruck bedingten, seitlichen Auslenkung und einer durch den Vorschub verursachten hinteren rückwärtigen und wechselhaften Auslenkung unterliegt.
Bei mikroskopischer Betrachtungistdie Abtragseffizienzdes Hubenswohlaufeinentieferen Eingriff der Diamantpartikel in die Werkstückoberfläche zurückzuführen, die nur deshalb möglich ist, weil das Huben einerseits durch die ständig helikale Bewegung eines jeden Schneidpartikels die Bildung eines kongruenten Schleif-Flächenreliefs zwischen Werkstück- und Werkzeug-Oberfläche verhindert und andererseits durch dieselbe makroskopische HubBewegung den Abtransport des Schleifstaubs mit gleichzeitig perkolierender mikroskopischer Kühlmittel-Zufuhr realisiert. Mit diesem Gedankenmodell ließen sich die zuvor erwähnten geringeren Umlaufgeschwindigkeiten, der höhere Anlagerungsdruck und die Laufruhe erklären.
Im Gegensatz hierzu findet bei dem eingangs erwähnten ULTRASONIC-Verfahren zweifelsfrei eine helikale Hartpartikel-Bewegung statt, jedoch nur im Mikrometerbereich und mit geringerer mikroskopischer Eingriffs-Tiefe. Dies erklärt die Effizienz dieses Verfahrens bei der Hartkeramik-Verarbeitung(gehipptesZirkon), Für die Rissbildung empfindlichen Glaskeramiken und Lithium-Di-Silikate stellt sich die Frage, in wie weit die hochfrequente Schwingung im primären Sinne eine Rissbildung fördern könnte. Aus der Zahnheilkunde ist bekannt, dass dieVerwendungvonTurbinenzur Präparation von Schmelz begrenzten Kavitäten zur Rissbildung und späteren Schmelz-Abplatzungen führen kann.
Die vertikalformende Bearbeitungkannals Hubenmit Seitenauslenkungbetrachtetwerden. So erklärt sich aus dem oben Gesagten die zunehmende Effizienz mit abnehmender Seitenauslenkung. Diegleichzeitighubende und Werkstückformende Bearbeitungistein Vorteil gegenüber dem ausschließlichen Huben. Wird das Huben jedoch mit einer simultanen tangentialen fünfachsigen Bearbeitung mit ständig angepassten Winkelwertenkombiniert,istes diesem überlegen (Abb.1 FDS II).
Weitere Merkmale und Ausstattungen bestehen darin, dass die Hubfrequenz 0,5 HZ bis 10 HZ beträgt; die alternierende Auf- und Abbewegung durch den Linearantrieb der Z-Achse erzeugtwird,wobei die Hubbewegung ausgehend von einem Werkstück bezogenen Koordinatenpunkt (Z=i) um den BetragderHubweglänge(h)zuerstaus dem Material heraus fährt (Z=i - h),um dann denselben Betrag wieder einzufahren (Z=i-h+h); die Hubfrequenz bei mit Linearmotor betriebenen Antrieben 2 bis 6 Hertz beträgt, bei mit Kugelspindel betriebenen Linearantrieben 0,5 HZ bis 3 HZ; die Hubweglänge der jeweiligen Eingriffshöhe der Werkzeugmantelfläche im Werkstück angepasst wird; die Hubweglänge 20% bis 50% der jeweil i-gen Eingriffshöhe der Werkzeugmantelfläche im Werkstück entspricht; die Hubweglänge und Hubfrequenz konstant sind und/oder mehrere konstante Hubsequenzen zur Verfügung stehen; die Hubsequenzen direkt in der CAM-Software-Strategie hinterlegt sind oder durch An- und Ausschalten (PLC) indirekt über die Steuerung abgerufenwerdenund/oderdurcheine externemechanische Hubvorrichtungim Sinneeinerkinematischen Überlagerungstattfindet; diemantelseitige Umfassungdes Werkzeugsim Werkstückgrößerals 90° ist (U > 90° bis 180°) (Bei seitliche Zustellung Sxy > 1r).

Besonders vorteilhaft ist es, gemäß Anspruch 2, die herkömmlichen horizontal und vertikal formenden Bearbeitungsverfahren in einer CAM-Strategie zu kombinieren. So ist beispielsweise die Schrupp-Bearbeitung der Seiten I und II mit eher horizontal ausgerichteten Oberflächen vorteilhafter Weise mit horizontal formenden Verfahren zu bewerkstelligen und die Bearbeitung der Seiten III bis VI, mit eher vertikal ausgerichteten Oberflächen im vertikal formenden Verfahren.

Beidiesem "beengtenvertikalen Schleifen" - hier BVSgenannt - ist es sinnvoll, die Mindestauslenkung der kreisenden Bewegung (x-y) im Bereich von 1/4, 1/3 bis 1/2 des Werkzeugdurchmessersanzulegen, um die verminderte Abtragseffizienz der Werkzeugstirnfläche zum Achsmittelpunkthinauszugleichen. Mitzunehmender Auslenkungvergrößertsichdas geschonte Stirnflächenareal (4), jedoch ebenfalls der Mindestdurchmesser der Kavität (Lmin).
In den Abbildungen 1e, 1f und 1h sind drei zylindrische Schleifer mit gerundeten Kanten unterschiedlicher Radien im Verhältnis ihrer Durchmesser skizziert. Die in den Linien (4) markierten zentralen geraden Anteile stellen die zu schonenden, achsennahen, sinusförmig markierten Anteile (5) derzum Substanzabtraggeeigneteren Werkzeugstirnflächedar. Durch Überlagerung der Linien der jeweiligen Positionen wird deutlich, dass die achsennahe Stirnfläche geschont wird. Betrachtet man den entstehenden Mindestdurchmesser des Lochs (Lmin), wird deutlich, dass kleine Kantenradien zu bevorzugen sind. Für größere Durchmesser ist dies zutreffend. Bei kleineren Werkzeugdurchmessern ist jedoch die Empfindlichkeit kleiner Radien im Hinblick auf die Diamantkorn-Verankerung zu berücksichtigen. Deshalb ist bei der Wahl der Form und des Werkzeugdurchmessers neben der Nutzung als Schrupp- oder Schlichtwerkzeug auch der gewünschte Lochdurchmesser und die Lochtiefe im Besonderen hinsichtlich der Kühlmittelzufuhr entscheidend. Bei der Bearbeitungderlnnenseiten von Kronenbesondersschmaler und paralleler Zahnstümpfe oder individuellen Abutments (künstliche Stümpfefür Implantate) oder Geschiebensindkleine Kantenradiengünstig. Der erwähnten Empfindlichkeitistdurchgeringe Vorschübeentgegenzuwirken. Zur Gewährleistung der effizienten Kühlung kann es zweckmäßig sein, das beengte vertikale Schleifen von beiden Seiten (Extrados und Intrados) her durchzuführen. Abbildung 1g veranschaulicht die EffizienzderWerkzeugneigung.EineNeigungvon nur 5° führt zur wirkungsvollen Schonung der achsennahen Stirnfläche und demonstriert gleichzeitig, dass die Kantenradien und damit die Standzeiten beim 4-5-Achs-Betrieb vergrößert werdenkönnen(5).Diesverdeutlicht, dass auch die ausschließlich hubende Bearbeitung von der Neigung des Werkzeugs besonders profitiert und sogar ein sanftes vertikales Eintauchen möglich ist, ohne das empfindliche Stirnareal(4).

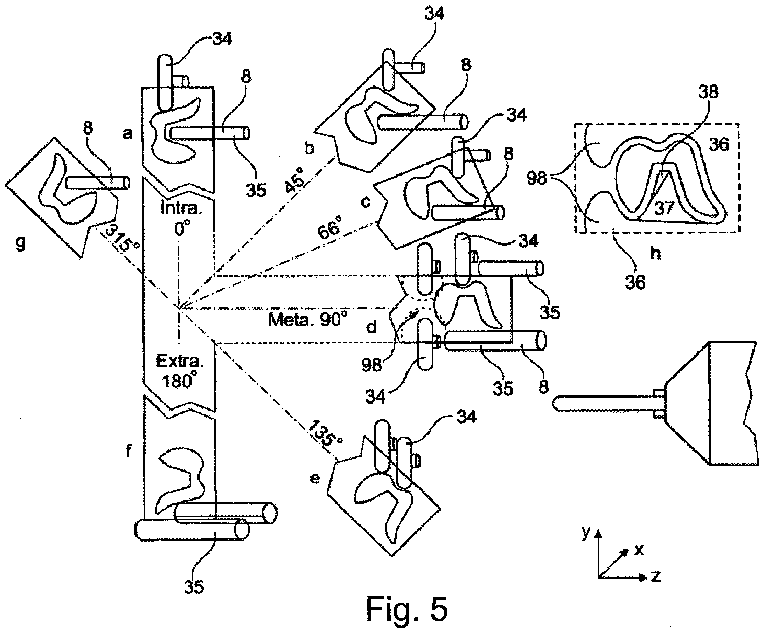

Generell ist das Einschwenken immer zu bevorzugen, wenn 4- oder 5-achsige Ausstattung vorliegt und wenn es die Werkstückgeometriezulässt, umdieseitliche Umfangsflächezu nutzenunddieachsennahe Stirnflächezuschonen. Deshalbistessinnvoll, insbesondere bei zunehmenden Kavitätendurchmessern, anstatt der kreisenden Bewegung das Eindringen ins Material auch mit noch so geringem Winkelvorzunehmen. Beiwenigerbeengten Kavitätenistesjedochvorteilhafter, das Eindringen (Einfahren) des Werkzeugsineinemmöglichst großen Winkel zwischen Werkzeugachse und Kavitäten-Achse (Kronen-Einschubrichtung) in einem Winkelbereich von 0° bis 90° vorzunehmen, beziehungsweise den zuvor beschriebenen Vorgehensweisen voranzustellen (siehe Abbildungen 5a bis 5d, 5g (8) und 5h (37,38).

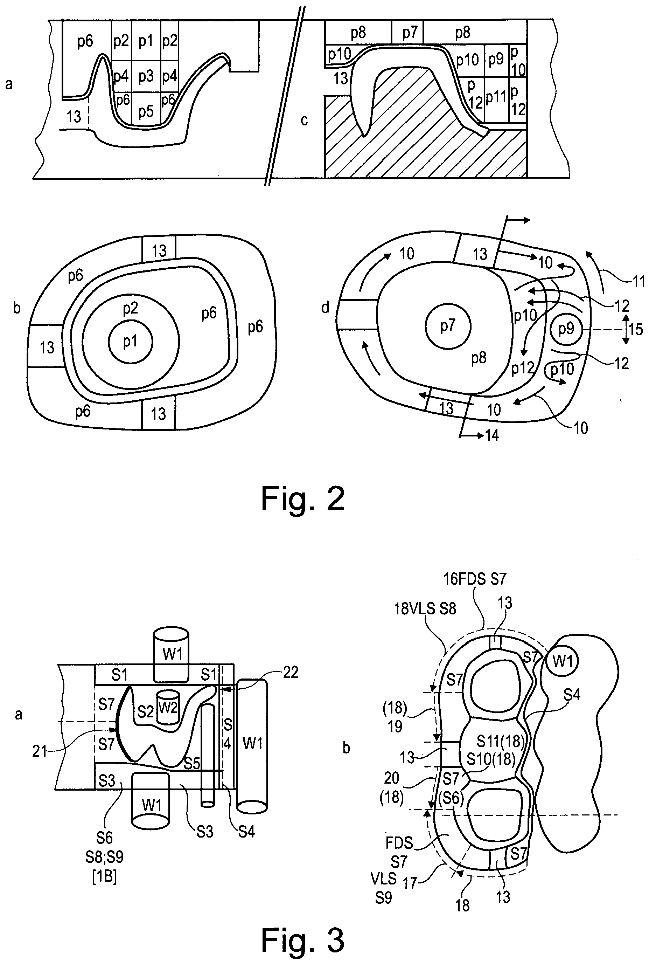

Ein vorwiegend horizontaler Materialabtrag erfolgt beim "vertikal limitierten Schleifen",hier VLS genannt (siehe auch Abbildungen 2, 3 und Erläuterungen). Als klassischer Fall ist beispielsweise die Extrados-Bearbeitung des Kauflächenabtrags in den Abbildungen 1b, 1d von Position VLS 2-5 anzusehen, bei dem mit alternierenden Bewegungen, oder mit hubender Unterstützung die Bearbeitung erfolgt . Im Eigentlichen ist jedoch der gesamte Bearbeitungsvorgang in den Abbildungen 1b, 1d von VLS 1 bis 5 als vertikal limitiertes Schleifen anzusehen(horizontalformendesVerfahren).Gleichesgilt für das zentrifugal spiralförmig (helikale) durchgeführte Schleifen (6) der Kroneninnenlumina nach vorheriger maximaler Z-Zustellung (BVS in Abbildung 1a bis 1d (3)) oder nach vorheriger schrittweiser Z-Zustellung, wie in Abbildung 2a dargestellt (p1, p3, p5 = BVS / p2, p4, p6 = VLS).

Eineweitere Schleifstrategieder Erfindungstelltdas "beengtedurchdringendevertikale Schleifen" (BDS) (7, 7.3, 7.4) dar. Bei kompletter Durchdringung des Rohlings in seiner Höhe (Dicke) mitentsprechendlangen Werkzeugarbeitsflächen findet gemäß dem oben beschriebenen Freihuben (7.3 ↔ 7.4) unter Nutzung der nahezu gesamten Umfangsfläche ein Material sparendes Freilegen oder Trennen statt. Da sich Umfangsfläche und abzutragendes Material mit dem Durchmesser proportional verhalten, (s.o.) können auch dünne Schleifer eingesetzt werden, mit beispielsweise 1,5 mm oder 2 mm. Allerdings ist es häufig sinnvoll, insbesondere bei Zunahme der Dicke den horizontalen Vorschub (7.1) nicht direkt geradlinig, sondern mit minimalem seitlichen Freischleifen (7.2) zu kombinieren.

Eine weitere besonders vorteilhafte Vorgehensweise stellt die Kombination der Hubbearbeitung bzw. des BDS mit gleichzeitigem formenden Schleifen - "formendesdurchdringendes Schleifen" (FDS) dar. Hierbei findet ein effektives Freischleifen durch zusätzliches Öffnen des ansonsten schmalen Schleifspalts bei dem durchdringenden Schleifen statt. In Abbildung 1a ist dies skizziert, wobei eine Querschnittsdarstellung vorliegt und die linke Bildseite die 31/2-achsige(FDSI - Extrados-Bearbeitung), die rechte Bildseite die 4-/5-achsige Bearbeitung (FDS II - Intrados-Bearbeitung) darstellt. Der Bewegungszyklus entspricht auf beiden Seiten 12344321 oder alternativ für FDS II 12344321-125521. Der Bewegungszyklus 12344321 entspricht dem tangentialen Schleifen mit kontinuierlich angepasstem Tangenten-winkel (%-Achs-Simultanbearbeitung). Bezüglich des hier Beschriebenen sei an die vorangegangenen Ausführungen zu Kombinationen des Hubens mit unterschiedlichen Z-Zustellungen erinnert.

Die Wirkung der Erfindung soll nun anhand eines weiteren Ausführungsbeispiels in Anlehnung an die herkömmliche Technik - horizotal formende Bearbeitung mit zu- und abnehmender Z-Zustellung-, wobei die zuvor genannten Schleifarten in einer kombinierten Schleifstrategie zur Anwendung kommen, erläutert werden, die gemäß Anspruch 9 mit dem Huben zu kombinieren sind. In Abbildung 2 ist das Schleifen eines Molaren-Käppchens skizziert, wobei die Skizzen 2a und 2b die Bearbeitung der Intrados-Seite, die Skizzen 2c und 2d die der Extrados-Seite und die Skizzen 2a und 2c den Querschnitt und die Skizzen 2b und 2d die Aufsicht darstellen. Die Bearbeitung beginnt auf der Intrados-Seite durch schrittweises beengtes vertikales Schleifen (p1, p3, p5) jeweils gefolgt vom vertikal limiterten Schleifen (p2, p4, p6). Letzteres (p6 / VLS) beginnt nach Erreichen der maximalen Z-Zustellung(p5)mit zentrifugal spiralförmiger Bewegung (6) mit ständig abnehmender Z-Zustellungbisüberdie Präparationsgrenze hinaus ins zirkumferente Freischleifgebiet mit zunehmender Z-Zustellung unter Freilassung etwaiger Stege bzw. Halter (13). Nach Wendung der Ronde erfolgt die Extrados-Bearbeitung. Diese beginnt mit dem vertikalen Eindringen im Zentrum der Kaufläche (p7 / BVS) gefolgt vom zentrifugal spiralförmig ausweitenden Abtrag (p8 / VLS). Im äußeren Randgebiet erfolgt ein weiteres vertikales Eindringen (p9 / BVS). Nun findet ein vertikal limitiertes Schleifen mit quer zum Vorschubvektor (10) ausgerichteter, alternierender Freischleifbewegung (9) statt (p10), wobei der Vorschubverlauf in einer Außenbahn (p10) beginnt und in einer oder mehreren weiteren Innenbahnen (p12) endet. Alternativ kann es zweckmäßig sein, die quer zum Vorschubvektor (11) ausgerichtete alternierende Freischleifbewegung (12) auf die gesamte Breite auszudehnen, sodass nur eine Bahn erfo r-derlich wäre (entspricht dem vertikalen Formen im Gegensatz zum üblicherweise praktizierten horizontalen Formen wie in der Einleitung beschrieben). Gleiches gilt für das stufenweise vertikale Eindringen (p9 und p11), welches auch in einem Schritt erfolgen kann. Zweckmäßig ist es daher, das stufenweise vertikale Eindringen (p9, p11) mit der weiten Freischleifbewegung (12) zu kombinieren, bzw. das vollständige Eindringen mit der Mehrbahn-Strategie (p10, p11) zu verbinden.

Eine weitere Möglichkeit besteht darin, zumindest in dem von den Haltestegen (13) eingeschlossenen Bereich (14) mit der Strategie des formenden durchdringenden Schleifens zu arbeiten. Dabei würden die Bearbeitungsfolgen p9 bis p12 ersetzt werden durch beengtes vertikales Schleifen mit anschließender Durchdringung zum Beispiel in der Position von p9 (Abbildung 1d) gefolgt vom formenden durchdringenden Schleifen (15, 14) bis zu den Stegen (13).

In Abbildung 3 ist ein weiteres Ausführungsbeispiel (Anspruch 3,4) der Erfindung skizziert, bei dem ein direkter Zugang zur Stirnfläche der Ronde durch eine zuvor geschliffene Brücke besteht. Die Teilskizze 3a zeigt den Querschnitt, die Teilskizze 3b die Aufsicht auf die Intrados-Seite, wobei die Bearbeitung im 31/2-Achsmodus stattfindet. Aufgrund des (indirekten) freien Zugangs wird ein Schruppschleifen mit großem Werkzeugdurchmesser -zum Beispiel von 4mm - gewählt. In chronologischer Reihenfolge erfolgt die Bearbeitung S1 (VLS), S2 (BVS>VLS), Rondenwende (180°), S3, S4 und S5. Der Bearbeitungsvorgang S5 kann mit vertikaler (FDS) oder wie hier skizziert mit horizontaler (VLS) Formgebung erfolgen. Sinnvoll kann es sein, die beiden Vorgänge im Schrupp- und Schlichtvorgang zu kombinieren. Hierbei ist jedoch auch die Komplexität der CAM-Software zu berücksichtigen. Anschließend folgt der ein- oder mehrstufige Schlichtvorgang (W2) an den genannten Flächen. Die Bearbeitung der noch ausstehenden Flächen S6 und S7 kann wie in den Erläuterungen zu Abbildung 2 erfolgen oder aber in einer weiteren vorteilhaften Modifikation. Hierzu wird sukzessive mittels vertikal formenden Schleifens - das heißt "formenden durchdringenden Schleifens (FDS)" - von der Intrados-Seite her die Bearbeitung S7 auf den Streckenabschnitten (16, 17) vorgenommen. Der jeweilige Haltesteg (13) wird mit abgetragen. Danach erfolgt die Bearbeitung der Abschnitte S8 und S9 (18) als Synonym und Anteil von S6 (Abbildung 3a) - im VLS-Modus in Extrados-Position. Dieser Vorgang wird in den Streckenabschnitten (19, 18) unter Auslassung des Haltestegs (20) durchgeführt, wobei die Abschnitte S10 und S11 dem von S6 (18) entsprechen. Siehe hierzu auch die anknüpfenden Erläuterungen zu Abbildung 6.

Besonders vorteilhaft ist es, die CAM Software derart auszustatten, dass vor dem "Nesting" und dem Setzen der Haltestege eine Vorberechnung der Restauration stattfindet, in der die abzutragenden Volumina bei der Bearbeitung im formenden durchdringenden Schleifen sowohl von der Extrados-Seite als auch von der Intrados-Seite (S7) her berechnet und verglichen werden und dann vollautomatisch oder manuell vom Nutzer die Seite der Bearbeitung zugunsten der mit dem größeren Volumen festgelegt wird. Das Volumen wird durch die Lage des Äquators (21, 22) bestimmt. In Abbildung 3a ist die Differenz der beiden Volumina auf der linken Bildseite (21) vergleichsweise gering. Auf der rechten Bildseite (22) ist die Abweichung dahingegen eklatant. Eine Bearbeitung von der Intrados-Seite an dieser Stelle im FDS-Modus käme einem beengten durchdringenden Schleifen gleich. Ein jegliches durchdringendes Beschleifen bedingt die Trennung vom Restmaterial. Es ist deshalb aus Gründen der Stabilität von Bedeutung nach einem durchdringenden Schleifen das Volumen der Nachbearbeitung (Abbildung 3a, S6, (8), S8-S11) möglichst gering zu halten.

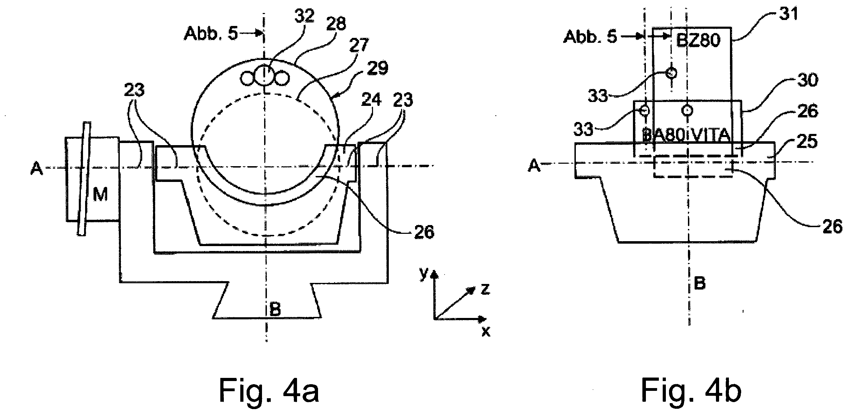

Die bisherigen Ausführungen verdeutlichen die Effizienz der Erfindung gemäß Anspruch 1. In Abbildung 4 sind zwei Ausführungsbeispiele der Werkstückbefestigung für die stirnseitige Bearbeitung aufgeführt.
Die Teilskizze 4a zeigt den weit verbreiteten Aufbau von 31/2-achsigen bzw. 4- oder 5-achsigen Maschinenausführungen, in denen der Werktisch beidseitig (23) in der A-Achse gelagert beweglich angeordnet ist. Üblicherweise umschließt der Werktisch die Ronde gegebenenfalls mit Halter umfänglich (nicht dargestellt). Die Teilskizzen 4a und 4b zeigen grob schematisch zwei Ausführungsformen von modifizierten Werktischen (24, 25) für die stirnseitige Bearbeitung, die zum Umrüsten vorgesehen sind. Bei ersterem (24) finden die klassischen Ronden Verwendung, die durch Klemmwirkung (26) fixiert werden. Die Anordnung kann wie üblich zentral erfolgen (27). Vorteilhaft ist es jedoch, die Ronde oberhalb der A-Achse anzuordnen (28), da so neben der Stirnfläche auch die Seitenflächen (29) der Bearbeitung zugänglich werden. Gleiches gilt für den Werktisch (25) in Teilskizze 4b, in dem rechteckige Rohlinge Verwendung finden, wie beispielsweise die im Handel erhältlichen VI-TA ZB80- oder AB80-Blöcke (Infiltrationskeramik), hier ohne metallischen Rahmen, die quer (30) oder hochkant (31) angeordnet werden können, wobei als Klemmfläche (26) ca. ¼ der Höhe des Rohlings fungiert. Die verbleibenden Reststücke der Rohlinge können über hinlänglich bekannte Einbettverfahren weiter genutzt werden. Die in den Rohlingen (29, 30, 31) dargestellten Restaurationen (32, 33) sind im Querschnitt in Teilabbildung 5a skizziert.

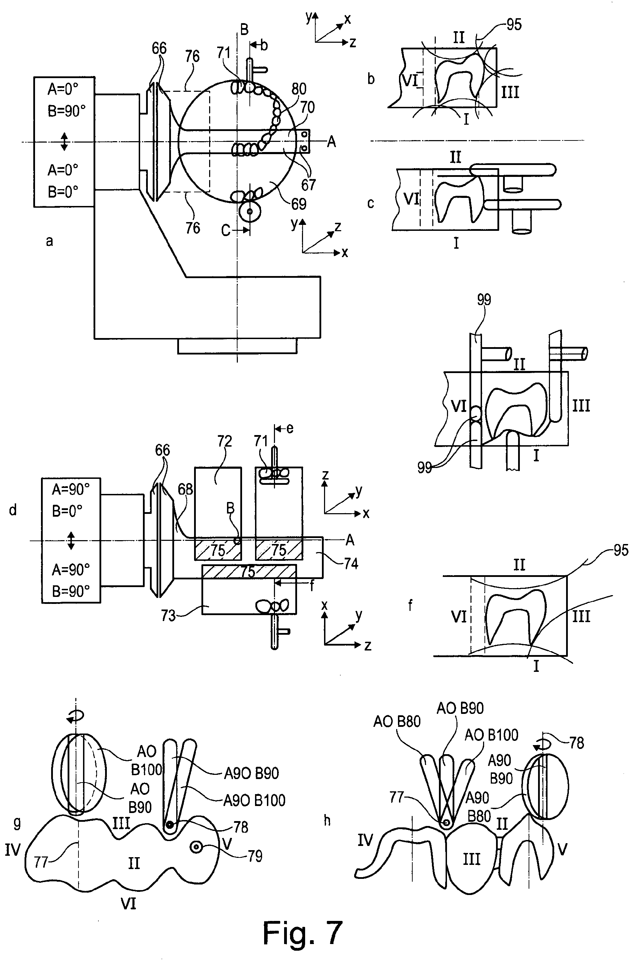

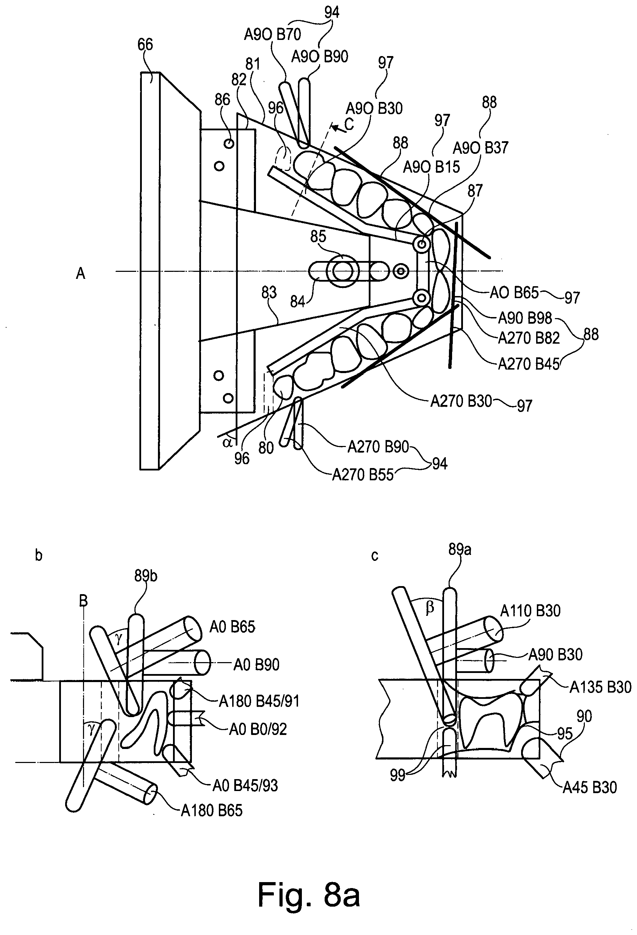

Schleifen oder Fräsen mit Walzen, Scheiben oder Linsen oder großkalibrigen Werkzeugen stellt eine weitere effiziente Bearbeitungsstrategie dar. Die Verwendung von Scheiben- oder linsenförmigen Werkzeugen ermöglicht hohe Umlaufgeschwindigkeiten bei vergleichsweise geringen Umdrehungszahlen, hoher Rundlaufgenauigkeit und damit extremen Substanz-Abtragungssraten, die nicht nur den Schruppvorgang beschleunigen, sondern auch bei insbesondere schmalen Ausführungsformen für den Vorgang des Schlichtens effizient eingesetzt werden können (siehe Abbildungen 7 und 8 (77, 78, 94, 95, 99)).

Betrachtet man in Abbildung 5 die drei Grundpositionen 0°, 90° und 180° (5a, 5d, 5f) mit den zylindrischen (35) und walzenförmigen (34) Werkzeugen, so lässt sich unter ausschließlicher Nutzung der seitlichen Umfangsfläche des Werkzeugarbeitsteils der in Abbildung 5h dargestellte Querschnittsabtrag (36) realisieren, dies sogar im 31/2-achsigen oder besser 32/3-achsigen Betrieb und zudem sogar schon im Schruppvorgang. Wenn nun noch die weiteren Positionen 5b, 5c, 5e (4-Achs-Betrieb) dazukommen, sind die Flächen (37, 38) des Kronenlumens dazuzuzählen. Ein weiterer wichtiger Vorteil ist darin zusehen, dass mit extremen Neigungswinkeln der A-Achse (insbesondere 315°, 45°) gearbeitet werden kann, da vor allem die Kollisionsgefahr mit dem Werkstück selbst und den wegfallenden Werktischanteilen beträchtlich reduziert wird.

Der generell freie Zugang von mindestens drei Seiten I, II, III ermöglicht den Einsatz großkalibriger Werkzeuge mit hohen Umfangsgeschwindigkeiten bei geringerer Drehzahl und gleichzeitig vergrößerter Umfangsfläche und damit eine erhöhte Standzeit der Werkzeuge. Ein weiterer wichtiger Wirkungseffekt stellt die Reduktion der durch die Bearbeitung entstehenden Schwingungen nicht nur in der Maschine sonder in dem Werkstück selbst dar. Dies insbesondere im Hinblick auf die Bearbeitung von Metallen in Form von High Speed Cutting. Hier werden hohe Umfangsgeschwindigkeiten bei geringerer Zustellung und höheren Verfahrensgeschwindigkeiten angewandt. Gleiches gilt ebenso für die Verarbeitung von Kunststoffen wie PMMA, Composites und andere.

Besonders vorteilhafte Wirkungseffekte ergeben sich für die Bearbeitung von Keramiken wie Glas-, Feldspat-, Infiltrationskeramiken und gehipptes Zirkon, die sich nur mit diamantierten Werkzeugen bearbeiten lassen. Der der Erfindung zugrunde liegende Wirkungseffekt, die nahezu vollständige Bearbeitung durch Nutzung der Umfangsfläche des Werkzeugarbeitsteils bei gleichzeitiger Schonung der achsennahen Stirnflächenanteile, kommt verstärkt zur Geltung. Die größeren Werkzeugdurchmesser und Umfangsflächen verbessern die Freischleifeigenschaften (siehe oben).

Ein weiterer wesentlicher und vorteilhafter Effekt ist, dass nicht nur die 5-Achsmaschinen sondern auch - oder eigentlich im Besonderen - die weit verbreiteten 4- und 31/2-achsigen Maschinen von diesem Wirkungseffekt der Erfindung profitieren. Letztere müssten dann 32/3-achsigbezeichnetwerden.

Die Gegenüberstellung der Abbildungen 1b und 5 demonstriert die Effizienz der Erfindung. Nahezu der gesamte Schruppvorgang lässt sich unter diesen Bedingungen extrem schnell und schonend bewerkstelligen. Weitere Erläuterungen ergeben sich aus der Zeichnungsbeschreibung.

In Abbildung 6a ist ein weiteres Ausführungsbeispiel eines Werkstückhalters gemäß Anspruch 12 für rechteckige Rohlinge (41) dargestellt (1:1-Maßstab). Der ZB 80 Blank (41) (Vita, s.o.) ist hierbei mit einem L-förmigen Rahmen (42) verklebt (43), der von dem zu verwendenden Werkzeug, hier Diamantschleifer, leicht bearbeitet werden kann (zum Beispiel Kunststoff, Leichtmetalle). Der Rahmen mit Werkstück wird in der Haltevorrichtung (40) durch Klemmwirkung befestigt, wobei der Mindestabstand zwischen Werkstück (hier: Keramik) und Haltevorrichtung so gewählt ist, dass das zirkumferente Schleifen (3 mm) im Rahmenmaterial stattfinden kann (44). Auf diese Weise gelingt es, bei stabiler Befestigung den Rohling ohne Materialverlust nutzen zu können. Die Haltevorrichtung (40) selbst wird wiederum mittels Adapter (45) durch Klemmwirkung am Werktisch befestigt. In diesem Ausführungsbeispiel lässt sie (40) sich quer (46) oder auch hochkant (47) am Werktisch fixieren und hat an ihrer Befestigungsseite die gleiche Abmessung in Höhe und Breite wie ihr Rohling [80 x 15 (46) / 40 x 15 (47)], sodass sie auch in anderen Haltern (25) für Vita-Blanks eingesetzt werden kann (siehe hierzu auch Abbildungen 8 bis10). Besonders geeignet ist diese Befestigungsart des Werkstücks auch für Glaskeramik- und Lithiumdisilikat-Rohlinge oder für Restmaterialien (30), natürlich mit angepassten Abmessungen der Rahmen und Halter. Zweckmäßig ist es, insbesondere bei der unilateralen Werktischbefestigung, einen Schenkel des Rahmens bzw. der Haltevorrichtung zur Seite der Aufhängung anzuordnen.

Ein besonders vorteilhafter Wirkungseffekt der Erfindung ergibt sich, wenn die Bearbeitung der dem Restmaterial des Rohlings zugewandten Seite erst nach Abschluss der fertig gestellten 5 Seiten erfolgt (siehe Anspruch 3). Dies wurde schon bei der Beschreibung der Abbildung 3 aufgenommen und soll nun anhand der weiteren Beschreibung der Abbildung 6 fortgeführt werden. Abbildung 6c stellt die Ausschnittsvergrößerung der Abbildung 6a dar. Die zu schleifende Restauration (48) ist an der Grenze zu dem schon verbrauchten Material (49) im Restmaterial (50) positioniert. Die sechs zu bearbeitenden Seiten sind mit römischen Ziffern beschriftet, wobei Seite I der Intrados-Kroneninnenseite, Seite II der Extrados-Kaufläche, Seite III der Metados-Seite (hier vestibulär) entspricht und die Seiten IV (hier: distal), V (hier: mesial) und VI (hier: lingual) die restlichen Seiten einnehmen, wobei Seite VI die zuletzt zu bearbeitende Längsseite darstellt. Diese Zuordnung der Seiten ist für die gesamte Beschreibung zutreffend. Die Teilskizzen 6a und 6c zeigen die Sicht auf die Kaufläche (Extrados), die Teilskizze 6d die Längsschnittdarstellung, die Teilskizzen 6e, 6f, 6g die Querschnittdarstellungen aus Teilskizze 6c. Da die Positionierung der dentalen Restauration (Nesting) in dieser Ausführung zweckmäßigerweise zur seitlich offenen Seite (51) (linke Bildseite) des Restmaterials erfolgt, sind generell die vier Seiten I, II, III und IV frei zugänglich. Die Positionierung der vorab (52) und nachfolgend (53) zu bearbeitenden Restaurationen (52, 53) ist in der Teilskizze 6a mit dargestellt und erfolgt in gleicher Weise und mit paralleler Ausrichtung zur A-Achse. Die Abfolge der Bearbeitung soll nun an einem Beispiel weiter erläutert werden. Es soll hier nochmals darauf hingewiesen werden, dass die Kombinationen der hier erläuterten Bearbeitungen mit dem Huben gemäß der Ansprüche 2 und 9 zeichnerisch nicht dargestellt sind. Sämtliche skizzierten Bearbeitungsverfahren der Abbildung 6, gegebenenfalls unter Ausschluss der Teilabbildung 6 h, sind erfindungsgemäß mit hubender Bearbeitung zumindest in unterstützender Form vorgesehen.

Die Bearbeitung beginnt in der Metaposition (90°) unter eines torusförmigen 5-mm-Schleifers mit 0,75-mm Kantenradius (54). In diesem Bearbeitungsschritt erfolgt das Schruppen der Seiten II, IV und I gemäß der Schleiflinie SL1. Es folgt das Beschleifen in der Intrados- (0°) oder Extrados-Position (180°) gemäß der Schleiflinie SL2, gegebenenfalls erweitert durch die Positionen 45° (55), 135° und 315° (56) gemäß der Schleiflinie SL3 (siehe auch Anspruch 8). Es ist ebenfalls sinnvoll, statisch sogar günstiger, in umgekehrter Reihenfolge vorzugehen. Die Bearbeitung der Seiten III und IV erfolgt gemäß SL2 mit sich anschließender Bearbeitung der Seiten I und II in Metados-Position nach SL1. Wenn die Maschinenausführung und CAM-Software dies ermöglichen, ist selbstverständlich die stufenlose Bearbeitung und gegebenenfalls der 5-achsige Betrieb zu bevorzugen (Anspruch 8). Sofern es die geometrischen Verhältnisse zulassen, ist es zweckmäßig, auch die Kronen lumina gemäß SL4 so weit wie möglich zu bearbeiten.

Nach Werkzeugwechsel wird mit einem endgerundeten 2,5-mm-Zylinder mit rauer Diamantierung das Grobschlichten aller vorbearbeiteten Seiten bzw. Flächen (I bis IV) durchgeführt. Dies kann wahlweise (CAM) in herkömmlicher Art der horizontalen Formgebung oder der vertikalen Formgebung erfolgen(Ansprüche 2, 3, 9). Sinnvoll ist es, in diesem Schleifgang die Bearbeitung der über die Präparationsgrenze hinausgehenden Anteile gemäß SL5 auch auf der 6. Seite (VI) zu vollziehen, um diese hinsichtlich der Präzision so wichtigen Anteile der Restauration schon vorab, einer in einem Schritt erfolgenden Feinausarbeitung zugänglich machen zu können (Einsparung von Werkzeugwechsel). Anschließend erfolgt die Freilegung der Seite V (hier mesial) mit demselben Werkzeug mittels formenden durchdringenden Schleifens (FDS) in der Intrados-Position (57 → 58, Abbildung 6g), gefolgt von der Nachbearbeitung in der Extrados-Position(Anspruch4).HieranschließtdiekompletteSchlichtung und Feinschlichtung der bis hier bearbeiteten Flächen mit feinkörnigen Diamanten (D = 2 mm / D = 1,5 mm und D = 1 mm).

Sofern die statischen Gegebenheiten es zulassen, ist es natürlich von Vorteil, bei der Bearbeitung der fünften Seite das formende durchdringende Schleifen in die sechste Seite (VI) auszudehnen (FDS/Intrados) (57 → 59) und auchihre Freilegungvondergegenüberliegenden Seite (FDS/Extrados) (60 → 61) her inklusive kehrseitiger Nachbearbeitung stattfinden zulassen. Werkstück-Materialeigenschaften, Vorschübe, Zustellungen und Anlagerungsdruck sind hier bestimmend und jeweils individuell abzustimmen. Die weitere Bearbeitung der sechsten Seite erfolgt alternierend im FDS-Modus (59 → 63, 61 → 62) gefolgtvon der abschließenden Reduktion des terminalen Stegs (64) bis zum Abtrennen und Herunterfallen der Restauration, die maschinenseits aufgefangen wird. Nun kann dieBearbeitungder nächsten Restauration (53) stattfinden. Sofern mehrere Restaurationen dentaler Werkstücke parallel zur A-Achse nebeneinander angeordnet sind, könnte die Bearbeitung der in einer Frontlinie (65) positionierten Objekte (48, 52) in einem Gang erfolgen. Hierbei ist jedoch die Gefahr einer Kollision in der Metados-Position zu berücksichtigen. Die dreigliedrige Brücke (53) kann dagegen erst nach abgeschlossener Bearbeitung der verblockten Kronen (52) erfolgen, dannaberineinem Gangmitderdreigliedrigen Brücke (48). Auf diese Weise können ganze Rohlinge sowohl in Form der klassischen Ronde, als auch in Form der rechteckigen Blanks in mehreren Frontlinien unterbrechungsfrei bearbeitet werden. Letztere bringen den Vorteil mit sich, dass bei der Bearbeitung im Hochformat bei einer Breite des Rohlings von ca. 40 mm Seitenzahnbrücken, die sehr häufig eine Länge von 30 bis 40 mm aufweisen, sogar fünf Seiten (I bis V) frei zugänglich sind und somit die Bearbeitungszeit weiter verkürzt werden kann und Werkzeugstandzeiten erhöht werden können. Bei abweichenden oder unpassenden Längenabmessungen kann auf die Anordnung im Querformat zurückgegriffen werden (siehe auch Abbildung 7g).

Die rechteckige Form auch großflächiger Rohlinge ist erfindungsgemäß zu bevorzugen. Dies gilt nicht nur für die individuell gepressten Oxid-Keramiken, sondern im Besonderen fürdie industriell als Plattenware verfügbaren Metalllegierungen Titan und Kunststoffe wie PMMA, Nylon, Kohlefaser, Teflon usw. Aus diesen lassen sich rechteckige oder trapezförmige flächige Rohlinge besonders materialsparend und kostengünstig (Wasserstrahlschneiden) herstellen.

Ein weiteres Ausführungsbeispiel der erfindungsgemäßen Bearbeitungsstrategie nach Anspruch 6 soll anhand der Abbildung 6h erläutert werden. In einem vertikal formenden Verfahren wird das Werkstück abschnittsweise bearbeitet wird, wobei die Abschnitte durch vier Tangentenlinien begrenzt werden, deren Tangenten-Winkel 45° betragen. Die Abschnitte mit eher vertikal zur Intrados- bzw. Extrados-Oberfläche orientierten Werkstückoberflächen (Seiten III bis VI) werden mit einer Werkzeugachsen-Neigung zur Intrados- bzw. Extrados-Oberfläche vorzugsweise 90° bearbeitet. Dies entspricht der klassischen bevorzugten Bearbeitung nach dem vertikal formenden Verfahren.
Die eher horizontal ausgerichteten Werkstückoberflächen der Seiten I und II lassen sich weniger effizient bearbeiten. Erfindungsgemäß werden durch Bearbeitung in der Metados-Position zuvor horizontal orientierten Seiten I und II zu vertikal orientierte Flächen umfunktioniert. Da sowohl die Kauflächen, als auch die Kronenkavitäten in der Metadosposition Unterschnittbereiche (160) verursachen findet die Bearbeitung sinnvollerweise in der angepassten Neigung von 30° bis 45°(161) statt. Die Tangentenlinie der zum Restmaterial (162) orientierten Seite (VI) werden in der CAM-Software mit der Bearbeitungsgrenze (163), die der gegenüberliegenden Seite III mit der Rohlingsoberfläche gelotet (164) verbunden. Die Vorschubbahnen (165) der Bearbeitungsseiten I, II und III sind dargestellt. Die Winkelangabevon 75° ermöglichtdie Nutzungdes Verfahrensmit Maschinenausstattungenohneseitlichoffene Ronde mit einem Mindestneigungswinkel von 25°.

Ein weiteres Ausführungsbeispiel der erfindungsgemäßen Bearbeitungsstrategie nach Anspruch 8 beinhaltet dass eine tangentiale Bearbeitung entlang der Tangenten-Linie mit Tangenten-Winkel zwischen 0°und 90° (Intrados) bzw. 90° und 180° (Extrados) in mehreren festgelegten Schritten wie in den Abbildungen 6 i und j skizziert. Die Bearbeitung findetmit parallel zur Werkstückachse ausgerichteter Vorschubbewegung statt. Einschonobenaufgeführterdurchdie Erfindungbewirkter Effektbestehtdarin, Werkzeuge in Form von Scheiben, Walzen oder Linsen einsetzen zu können. Eine einfache, aber effektive Anwendung des Scheibenschleifens stellen die in den Abbildungen 4a und 4b dargestellten Werkstückanordnungen und deren Einsatz gemäß der Abbildungen 5 a, 5d und 5e dar (34).

DieWirkungsbandbreitelässtsicherweitern,wenn die Anordnungdes Werkstücksunddie Freiheitsgrade seiner Positionierbarkeit gegenüber dem Werkzeug beispielsweise der in Abbildung 7 skizzierten Ausführung entsprechen. Es liegt ein klassischer 5-Achsaufbau vor, das heißt A-und B-Achse kreuzen sich im Werkstückmittelpunkt. Zweckmäßig ist es, dass Werkstück einseitig an der Wendeachse, hier A-Achse, zu befestigen. Notwendig ist es dagegen, dass die B-Achsrotation, ausgehend von der Grundposition, definiert als B = 0°, mindestens 90° beträgt. Hierdurch entstehen zwei weitere Grundpositionen B 90-A 0 und B 90 A 90. (Anspruch 5) In der B = 90°-Postion ist die A-Achse parallel zur Werkzeugrotationsachse ausgerichtet. Die in den Abbildungen 7a und 7d skizzierte Maschinenausführung ist weiterhin mit einem Werktisch-Wechselsystem (66, zum Beispiel 3R-System) ausgestattet. Die Skizze 7 astelltdie Frontansichtbei A = 0° und die Skizze 7 ddie Aufsichtbei A = 90° dar. Dabei weisen die jeweils durch die A-Achse getrennten oberen und unteren Bildhälften unterschiedliche B-Achseinstellungen auf. Dies ist im Bereich des A-Motors deutlich markiert, unterstütztdurchkleine Koordinatensysteme. Jededervier Positionsvariantenistmiteiner Querschnittsdarstellung 7b, 7c, 7e, 7f ergänzt.

Eine besonders vorteilhafte Ausführungsformder Erfindungstellendie Werkstückhaltermit jeweils zwei freizugänglichenStirnflächen(67,68)dar.DieSkizze7azeigteineklassische Ronde (69), die in ihrer Mitte durch Klemmwirkung starr befestigt wird (67). Die von fünf Seiten freizugängliche Ronde erlaubt ein ständiges Arbeiten in Frontlinien (siehe oben) mit mindestens vier frei zugänglichen Seiten des dentalen Werkstücks (71) bis zum vollständigen Verbrauch des Rohlings, abgesehen des von den beispielsweise 15 mm breiten Klemmbacken (70) bedeckten Materials. Die Weiterverarbeitung dieses Restrohlings von 15 mm x 100 mm Fläche wurde oben schon erörtert (siehe aber auch unten). Gleiches gilt für die Darstellung in 7d, wobei hier der Werktisch bzw. Werkstückhalter (68) mit rechteckigen Rohlingen in Hoch- (72) oder Querformat (73) bestückt ist und deshalb die Werktischmitte mit Klemmvorrichtung (74) von der A-Achsmitte abweicht. Auch hier kann das von der Klemmvorrichtung bedeckte (75) Material der Weiterverarbeitung zugeführt werden. Da die Gesamthöhe bei dieser Anordnung 130 mm nicht überschreitet, üblicherweise ist der Durchmesser der Ronde 100 mm zuzüglich der üblichen 2 x 15 mm Werktischwand - lässt sich dieser Werkstückhalter in den meisten Maschinen einsetzen. Ein weiterer Vorteil besteht bei dieser Anordnung darin, dass bei der Positionierung (Nesting) je nach Abmessung der zu bearbeitenden Restaurationen die günstigste Position in einer der drei Rohlinge gewählt werden kann, um auf diese Weise unnötigen Materialverbrauch zu vermeiden.

In Abbildung 7a ist eine weitere Ausführungsform eines Werkstückhalters (67) aufgeführt, dessen Klemmvorrichtung (76) - mittels gestrichelter Linie skizziert - die Ronde (69) nur an einer Seite fixiert. Bei dieser hinlänglich bekannten Befestigungsart ergibt sich zumindest bei der eingangs genannten Maschinenanordnung der vorteilhafte Effekt, dass drei frei zugängl i-che Stirnflächen (-Seiten) der Ronde bearbeitet werden können.

Abbildung 7c veranschaulicht Bearbeitungsmöglichkeiten mit Scheiben oder Walzen in der Position A0-B0. Demnach werden die Seiten I bis III erreicht, in einer jedoch ungünstigen Position für eine formende Bearbeitung. Auch in der A90-B0-Position (7e) können diese Seiten bearbeitet werden. Darüber hinaus wird jedoch auch Seite IV und besonders Seite VI erreicht. Die Teilskizzen 7b und 7f sind sehr ähnlich und ermöglichen ebenfalls die Vorbearbeitung der Seiten I bis III und veranschaulichen, dass auch bei extremen Werkzeugdurchmessern (7f) die Effizienz der Ausarbeitung wenig leidet. Der Unterschiedzwischendiesen anscheinend so ähnlichen Bearbeitungspositionen besteht darin, dass in der A0-B90-Position (7b) eine Rotation der B-Achse beispielsweise um +/- 10° über eine orovestibuläre (bukolinguale) Restaurationsachse (77) oder Metados-Achse oder die Verbindungsachse zwischen den Seiten VI und III stattfindet. In der A90-B90-Position steht diese Schwenkachse (78) dagegen senkrecht zu ersterer und parallel zur Einschubrichtung der Restauration (79). Abbildungen 7g und 7h veranschaulichen diese Achslagen, wobei Abbildung 7g die Aufsicht auf die Extrados-Fläche und 7h die stirnseitige Aufsicht darstellt.

Eine besonders effiziente Bearbeitungs-Strategie für den Einsatz von Scheiben-, Walzen- oder Linsenförmigen Werkzeugen stellt das folgende Ausführungsbeispiel nach Anspruch 7 dar. In einem ersten Schritt erfolgt eine Bearbeitung des Werkstücks mit möglichst parallel zur Längsachse der zu schleifenden Restauration ausgerichteter Werkzeugachse. In Abbildung 7 a gilt dies für das Werkstück (71) in A 0-B 90-Position, in Abbildung 7d für das Werkstück (73) A 90-B 90-Position. Die entsprechenden Querschnittsdarstellungen 7b und 7d veranschaulichen die vorteilhaften Effekte. Die Bearbeitung erfolgt durch Abzeilen der Werkstückkontur gemäß einer alternierenden Vorschubbewegung (95) von den Seiten I zu III zu II und danach in umgekehrter Reihenfolge bei einer parallel zur Längsachse der Restauration angelegten Zustellung. Es besteht aber auch die Möglichkeit, Vorschubbewegung und Zustellung auszutauschen, wobei eine alternierende Vorschubbewegung entlang der Werkstücklängsachse und eine schrittweise Zustellung von Seite I zu III zu II stattfände. Es soll an dieser Stelle nochmals an die Möglichkeit der Kombination mit Hubwirkung gemäß Anspruch 9 erinnert werden, die auch bei scheibenförmigen Werkzeugen sinnvoll ist, sowohl im Längsais auch im Quereinsatz der Scheibenebene. Gefahren der Kollision kann durch Abwinkelung der Werkzeugachse einfach entgegengewirkt werden (Abb. 7g, h insb. Abb. 8). In einem zweiten Schritt erfolgt die Bearbeitung in der A 90-B 0 Position mit optimalen Bedingungen, ansonsten ungünstig zu erreichende Stellen, einfach bearbeiten zu können (Abbildungen 7e, 8b und c). Auch hier kann die Bearbeitung in parallel oder quer zur Werkstückachse erfolgen.

Den gesamten Kiefer umspannende Restaurationen (80), wie 14-teilige Brücken, Teleskop- und Stegbrücken und größere Implantatversorgungen, beschränken aufgrund ihrer zahnbogenförmigen Krümmung eine stirnseitige Bearbeitung. Nur die im unmittelbaren Randgebiet des Rohlingspositionierbaren Anteile der Restaurationlassensichstirn seitigbearbeiten. Zweckmäßig ist es daher, die randfernen Anteile gemäß der konventionellen Technik zu bearbeiten und damit die Verfahren zu kombinieren. Des Weiteren kann dieser Problematik durch Verwendung kleinerer, dem Zahnbogen angepasster Ronden-Durchmesser entgegengewirktwerden.

Eine besonders vorteilhafte Ausführungsformeiner solchangepassten Rondestelltderin Abbildung 8 dargestellte trapezförmige Rohling (81) mit einer Basis von 80 mm, einer Höhe von 60 mm und einem Schenkelwinkel α = 67° dar, deralsmittlere Größe angesehen werden kann. Seine Befestigung erfolgt mittels Klemmwirkung an seiner Basis (82) und eines trapezförmigen Klemmbereichs (83) in seinem Zentrum. Er enthält in seiner Mittellinie ein Schlitzloch (84) zum Durchlass der Klemmbackenschraube (85). Seine Positionierung erfolgt unter Berücksichtigung der geometrischen Form der zu schleifenden Restauration nah zur Befestigungsbasis (66), indem über Positionsstifte (86) stufenweise reproduzierbare Befestigungspositionen eingenommen werden können. Alternativ besteht die Möglichkeit, auf die Langschlitzbohrung zu verzichten und die Klemmwirkung schraubstockartig nur im Randgebiet gemäß den Linien (82, 83) stattfinden zu lassen.

Die trapezförmige Form ermöglicht das Erreichen der Flächen I bis III über den gesamten Zahnbogen (Restaurationsbogen) und damit seine stirnseitige Bearbeitung. Es sind verschiedene Bearbeitungsformen zu unterscheiden, die anhand eines Ausführungsbeispiels einer Bearbeitungsfolge für Metalle erläutert werden sollen. Der Vorgang beginnt aus statischen Gründen mit Bohrungen oder beengtem vertikalen Fräsen der Kronenlumina und Verbindungsbohrungen (87). Es folgt das schnelle Schneiden (Anspruch10) (Rapid Cutting) (88) mit dünnen Sägescheiben für die Metallbearbeitung mit Durchmessern von 30 mm (6-Schaft, Schnitttiefe bis 12 mm) bis 60 mm (8-Schaft, Schnitttiefe bis 26 mm). Bei einer Höhe des Rohlings von 20 mm ist der Einsatz einer 52-mm-Scheibe mit 8mm Schaft und einer maximalen Eindringtiefe von 22 mm ausreichend. Bei beidseitiger Bearbeitung genügt eine 30-mm-Scheibe mit 6-mm-Schaft und einer Eindringtiefe von 12 mm.

Zur weiteren Schrupp-Bearbeitung der Flächen I bis III werden dickschaftige Scheiben oder Walzen mit zylindrischer, kantengerundeter (89a) bis endgerundeter (89b, 34, 90) Querschnittsform, Durchmessern um 30 mm und Scheiben-Dicken von 3 bis 6 mm eingesetzt. Mit senkrecht zum Zahnbogen angeordneter Rotationsachse bei alleiniger Nutzung der Umfangsfläche des Werkzeugs findet die Bearbeitung dieser Flächen (I bis III) mit parallel zum Zahnbogen ausgerichteter Vorschubbahn und schrittweise angepassten A-Achswinkel im Seitenzahngebiet (90) statt, besonders anschaulich in Abbildungen 5a bis 5g (34, 36) wie auch in den Abbildungen 7c, 7e und 8c (90) dargestellt. (Anspruch 7)

Im Frontzahngebiet (Abbildung 8b) findet die Winkelanpassung mittels B-Achseinstellungen (91, 92, 93) statt. Zur weiteren Veranschaulichungdieses Bearbeitungsschrittslassensich auch die Darstellungen in den Abbildungen 6c und 6d heranziehen. Hierbei ist der torusförmige Schleifer (54) durch den Walzen oder Scheibenfräser zu ersetzen. Die Schleiflinie SL1 entsprichtdannder Bearbeitung der Flächen I und II, die Schleiflinie SL2 der Bearbeitung der Fläche III. Die Abbildungen 8b und 8c veranschaulichendiezusätzliche Möglichkeit, das Werkstück gemäß dem Winkel β mit 20° und dem Winkel γ mit 25° zu schwenken. Dies ist im Besonderen hinsichtlichder Kollisionsgefahrvon Bedeutung.

Hiernach erfolgt ein Nachschruppen derselben Flächen mit parallel zum Zahnbogen ausgerichteter Werkzeugrotationsachse gemäß den Abbildungen 8a (94), 7b und 7f (95). Hierzuist es zweckmäßig, dünnere Scheiben (Walzen) einzusetzen, um schon in dieser Phase der Vorbearbeitungeinefeinere Ausarbeitunginsbesonderederschmalen Zahnzwischenräume realisieren zu können. Des Weiteren kann in dieser Phase auch die Freilegung der Seiten IV respektive V stattfinden (96). Es folgtdasmehrstufige Schlichtender Seitenlbis V.

Die abschließende Bearbeitung der Seite VI kann ebenfalls mit scheiben- oder walzenförmigen Werkzeugen stattfinden (97). Dies kann einerseits in Art der durchdringenden Bearbeitung - zurVermeidungzu großer Werkzeugdurchmesser ist die beidseitige Bearbeitung vorzuziehen (99, Abbildungen 7e, 8c) - oderandererseitsdurchvertikalbegrenztes Eindringen (98, Abbildungen 5d, 5g) unter Auslassung von Haltestegen oder Haltebändern stattfinden. Der nachteilige Effekt des Scheibenschnitts insbesondere bei kleinen Zahnbogenradien (Frontbogen-Innenseite) kann durch Verbindungsbohrungen (87) reduziert werden. Eine Nachbearbeitung im klassischen Bearbeitungsverfahren mit Radius-Fräsern oder Torus-Fräsernistunerlässlich. Abschließend erfolgt das Schlichten der Seite VI. Zweckmäßig bei einer derartig umfangreichen Restauration ist es, mindestens drei Haltestege zu belassen und auf deren Abtrennung zu verzichten, zumal der Rohling zumindest in dieser Einspannungverbraucht ist. Die Weiterverwertung des Restmaterials kann über reproduzierbare Befestigung im Bereich des Schlitzlochs (87) mittels entsprechender Klemmvorrichtung, also mit umgekehrter Orientierung, stattfinden.

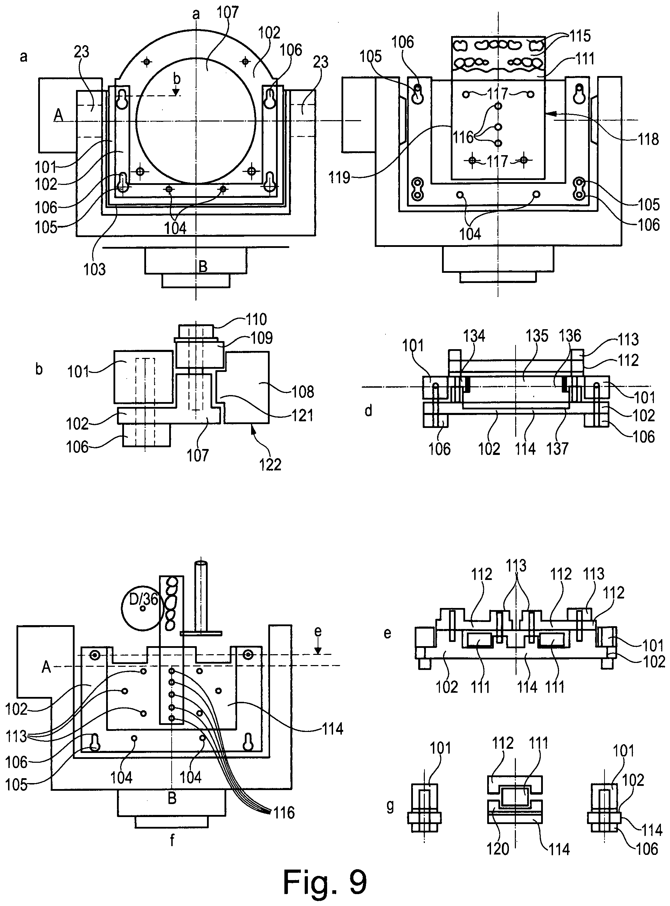

Wie schon in den Erläuterungen zu den Abbildungen 4a und 4b beschrieben, ist in Abbildung 9 ein weiteres Ausführungsbeispiel gemäß Anspruch 11 eines Werktischs mit beidseitig beweglicher Lagerung (23) für die Umrüstung bestehender Maschinen mit geschlossenen Werktischen aufgeführt. Dieser setzt sich aus einem U-förmigen Werktischrahmen (101), der beidseitig (23) mit den A-Achslagern fest verbunden ist, und einem auswechselbaren, durch Schraubverbindung zu befestigenden Werktischeinsatz (102) zusammen. Zum Stabilitätsausgleich ist der Rahmen in seinem Randgebiet (103) verstärkt worden. Ein jeglicher Werktischeinsatzistmitzwei Kugelschnappern oder Repositionstiften (104) ausgerüstet, dieindie entsprechenden Positionsbohrungen am Werktischrahmen einrasten, sodass eine exakte Reponierbarkeit aller Werktischeinsätze gewährleistet ist. Ihre Einbringung erfolgt- bei horizontal gelagerter Spindel - von vorne durch Schraubkopfdurchlässe (105), Absenkung bis zum Einrasten der Kugeln (104) und anschließendem Anziehen der vier Befestigungsschrauben (106). Zweckmäßig ist es dabei, diese Einbringung in der Metados-Position (A = 90°) vorzunehmen, um Schwerkraft bedingte Ungenauigkeiten zu vermeiden.

Die Abbildungen 9a und 9b zeigen den geschlossenen Werktischeinsatz (102) für das direkte Einbringen einer klassischen Ronde (107), die per Klemmring (109) und Spannschraube (110) befestigt wird.
In Anbetracht der einfachen und damit kostengünstigen Konstruktion des Werktischeinsatzes ist es sinnvoll, die Ronden bis zum vollständigen Verbrauch in diesem zu belassen und für jede weitere benötigte Ronde in einem eigenen Werktischeinsatz vorrätig zu haben. Es kann aber auch sinnvoll sein, den Werktischeinsatz in seinem Lumen als exakte Kopie des vorherigen, auszutauschenden Werktisch-Lumens zu gestalten, um beispielsweise die vorhandenen Werkstückadapter, Klemmringe und Distanzhalter weiter nutzen zu können. So kann in gewohnter Weise gearbeitet werden und im Bedarfsfall andere Werktischeinsätze verwendet werden.

Ebenso ist es zweckmäßig, die in den Abbildungen 7, 8 und 10 dargestellten Maschinenausführungen mit einseitiger Werktischbefestigung und Schnellwechselsystem (66) mit dem hier beschriebenen Werktischrahmen auszustatten, sodass sämtliche Werktischeinsätze auch in diesen Maschinenausführungen eingesetzt werden können (nicht gezeichnet).

Die Abbildungen 9c bis 9g zeigen einseitig offene Werktischeinsätze für quaderförmige Rohlinge (111). Die Werkstückbefestigung erfolgt durch jeweils eine Klemmplatte (112), die mittels Schraubkraft (113) an dem als Grundplatte (114) fungierenden Werktischeinsatz befestigt ist.
Ein wesentlicher Vorteil dieser Ausführungsform (Anspruch16) besteht darin, dass ein großflächiger Anteil des Werkstücks (111) in der Klemmvorrichtung (112, 113, 114) fixiert ist und nur der zur Bearbeitung notwendige Anteil von fünf Seiten frei zugänglich ist. Auf diese Weise wird eine hohe Befestigungsstabilität und Schwingungsarmut bei gleichzeitig maximal freiem Zugang realisiert.
Dies ist insbesondere bei der Bearbeitung von festen und harten Materialien wie Titan und kobaltchromhaltigen Metallen und von vergleichsweise elastischen Materialien wie PMMA und Nonocompositesvon Vorteil. Nach Verbrauchdesfreizugänglichen Werkstückanteils (115) wird nach Lösung der Klemmwirkung das Werkstück in definierten Schritten beispielsweise über einzelne (116) oder paarweise (117) angeordnete Positionsstifte nach oben verschoben und wieder geklemmt. Die definierten Verschiebungsbeträge sind in der CAM-Software einzugeben. Des Weiteren ist es sinnvoll, insbesondere bei größeren Werkstücken, wie in Abbildung 9c (zum Beispiel 120 mm x 80 mm x 20 mm), zur Unterbindung von Verkantungen vor der Wiederbefestigung eine einseitige Kraftbeaufschlagung durch Schraub- oder Federwirkung (118) zu erzeugen, um auf der gegenüberliegenden Seite (119) eine kongruente Anlagerung zu gewährleisten.

Ein besonders vorteilhaftes Ausführungsbeispiel stellt die in den Abbildungen 9f und 9g skizzierte Anordnung dar. Neben den zuvor genannten Vorteilen der hohen Stabilität und des freien Zugangs ist hier zusätzlich ein umfänglich großes Platzangebot für die Bearbeitung vorhanden. So können großkalibrige Scheiben- oder walzenförmige Werkzeuge (hier: D = 36 mm) eingesetzt werden. Dies ist besonders vorteilhaft für die Bearbeitung von Metallen, Glaskeramiken und Lithiumdisilikat-Keramiken. Ebenso ermöglicht die Verwendung von vergleichsweise günstigen Stangenmaterialien (Titan, CoCr-Stählen) das Anlegen vieler Sortimente unterschiedlicher Abmessungen der Rohlinge. Auch die oben erwähnten Restmaterialien lassen sich gegebenenfalls nach Vorbearbeitung einsetzen, wie auch sämtliche Rohlinge des Cerec- und Celay-Systems. Dies gilt gleichermaßen für die in Teilabbildung 9e skizzierte Anordnung. Um eine Zentrierung der Rohlinge bezüglich ihrer Lage zur A-Achse realisieren zu können, ist es zweckmäßig, Adapter (120) einzusetzen, die auf der Grundplatte (114) anzubringen sind. Ausgehend von der hier gewählten Querschnittsmaximalgröße von 20 mm x 20 mm erfordert ein gewünschter Rohling mit der Abmessung 20 mm x 12 mm einen Adapter mit einer Stärke von 4 mm.
Eine besonders vorteilhafte Ausführung nach Anspruch 13 einer Werkstückbefestigung ist in den Abbildungen 9 h und i skizziert. Hierbei erfolgt eine sekundäre Befestigung zwischen Werkstück (41) und dem Werktisch (46) über den Werkstückhalter (45) und Werkstückrahmen (42) erfolgt und die primäre Befestigung des Werkstückmaterials mittels einer ebenfalls am Werktisch (46) befestigten abnehmbaren Klemmvorrichtung, die sich aus einer Grundplatte (180) mit Befestigungsschrauben (183) und einer Konterplatte (181) mit Klemmschrauben (184) zusammensetzt und den Zugang (182) zu der sekundären Klemmvorrichtung(40) auslässt, stattfindet, wobei bei der Aktivierung der primären Klemmwirkung (185) vorzugsweise die geringfügige Aufhebung der sekundären Klemmvorrichtung zur Vermeidung von Spannungen stattfindet und in umgekehrter Reihenfolge nach Abschluss des Ersten und Beginn des zweiten Bearbeitungsschrittes.

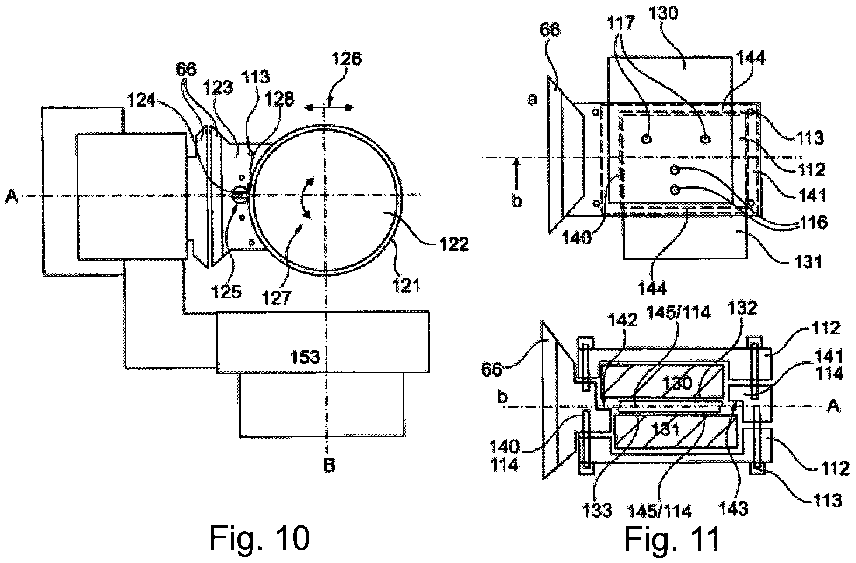

Abbildung 10 zeigt eine einseitig durch Klemmwirkung befestigte Ronde.Sieähneltderin Abbildung 7a beschriebenen Ausführung (76) mit dem Unterschied, dass ihre Befestigung unter nahezu vollständiger Auslassung der zu bearbeitenden Rohlingsfläche (122) ausschließlich in ihrem Randgebiet (121) stattfindet (siehe hierzu auch Abbildung 9b). Somit kann die Ronde bis zu ihrem vollständigen Verbrauch nahezu ohne Materialverlust und ohne anfallende Restmaterialien verarbeitet werden. Gemäß den vorangegangenen ausführlichen Erläuterungen zu den Abbildungen 7 und 8 und Nutzung der B = 90°- Positionen kann das Werkstück sogar vollständig in der der Erfindung zugrunde liegenden stirnseitigen Bearbeitungsweise von allen drei frei zugänglichen Seiten (oben, unten, rechts in Bildbetrachtung) bis zu seinem vollständigen Verbrauch ohne jeglichen durch die Befestigung bedingten Materialverlust verarbeitet werden. Gleiche Vorteile ergeben sich natürlich auch für die konventionelle Bearbeitungsweise. Besonders vorteilhaft ist es, die Ronde mit einer Repositionshilfe innerhalb des Befestigungsbereichs auszustatten. Dies kann in einfachster Weise dadurch erfolgen, dass die Ronde an der Stirnfläche ihres 10 mm hohen und 2 mm vorspringenden Absatzes (121) mit einer vertikalen, beispielsweise U- oder V-förmigen Kerbe (128) versehen wird, in welche das kongruente Gegenstück der Klemmvorrichtung (123) im Bereich der Achse in Form eines Keils (124) greift. Hierfür ist es erforderlich, diesen Bereich in der Klemmvorrichtung frei zu lassen (125) und damit einer visuellen Kontrolle zugänglich zu machen. Die Funktion dieser Repositionshilfe besteht in der Vermeidung von Verschiebungen in Richtung der A-Achse (126) und Rotationen um die Lotachse der Ronde (127). Eine präzise Wiederbefestigung des Rohlings im Sinne einer etwaigen Rotation um die A-Achse erfolgt durch die Klemmwirkung von selbst. Auf diese Weise ist es möglich, mit nur einer Klemmvorrichtung Ronden beliebig mit exakter Reponierbarkeit auszutauschen und auf die kostenintensiven Schnellwechselsysteme oder auf den Einsatz mehrerer Klemmvorrichtungen verzichten zu können. Die Anbringung einer Repositionhilfe kann auch additiv erfolgen, indem beispielsweise ein vorgefertigter Rundstab aus transparentem PMMA-Kunststoff in besagter Position mittels einer Hilfsapparatur und Licht härtendem Kunststoff an die Stirnfläche des Rondenabsatzes geklebt wird.

Diese Art Ronden-Befestigung eignet sich auch für die in Abbildung 4a dargestellte Ausführung und auch in Kombination mit den Werktischeinsätzen (102) in bilateral gelagerten Ausführungen.

Das in Abbildung 11 grob skizzierte Ausführungsbeispiel eines Werktisches bzw. Werkstückhalters mit Wechselsystem leitet sich aus der beschrieben Ausführung der Abbildungen 4c und 4d ab. Im Rahmen der hier aufgeführten einseitigen Werktischbefestigung besteht die vorteilhafte Möglichkeit, zwei Werkstücke (130, 131) nach demselben Wirkungsprinzip in einem Halteranzuordnen. Sokönnenbeispielsweisezwei Rohlingeunterschiedlicher Dicken in einer Aufspannung bearbeitet werden. Dies ist dahingehend von weiterem Vorteil, dass insbesondere kleineren Betrieben, die ständig wechselnde Materialien und Ronden-Stärken benötigen, ein größeres Angebot an Werkstück-Variationen zur Verfügung steht. Hierbei ist jedoch zu berücksichtigen, dass eine Zentrierung eines Rohlings in der A-Achse nicht sinnvoll erscheint. Es ist daher zweckmäßig, die Zentrierung der Grundplatte (114) in der A-Achse vorzunehmen und die dieser aufliegenden Werkstückoberfläche als Intrados-Oberfläche (132,133) zu definieren und in der CAM-Software zu berücksichtigen. Die Distanz der Werkstückmitte zur Achse errechnet sich aus der Hälfte der Grundplatten-Dicke und der jeweiligen Werkstück-Dicke. Die Skizze 11b ist für später erfolgende Erläuterungen modifiziert worden. Für die hiesige Beschreibung ist die Grundplatte (114) als ein Teil zu betrachten (114 = 140+141+145).

Ein besonders vorteilhaftes Ausführungsbeispiel ergibt sich, wenn die beiden Werkstückplatten in direktem Kontakt miteinander stehen, wobei deren Kontaktfläche (136) die A-Achse enthält und als Indrados- oder Ertrados-Oberfläche definiert werden kann. Dies ist durch die Modifizierung der Querschnittsdarstellung in Abbildung 9d veranschaulicht. Der dort dargestellte Rohling (111) wird durch die zwei Rohlinge (134, 135) ersetzt. Der obere Rohling (135) kann in seiner Dicke frei gewählt werden, da er keinen Einfluss auf die Zentrierung der Kontaktfläche (136) hat. Dagegen ist die Dicke des unteren Rohlings (134) gemäß der Grundplattenabmessung festgelegt. Es ist deswegen zweckmäßig die Grundplatte (114) derart zu gestalten (137), dass sie für die Aufnahme eines bestimmten Rohlings, beispielsweise des mit der maximalen Dicke (20 mm) oder des am häufigsten vorkommenden (10 mm), vorgesehen ist. Im ersteren Fall besteht bei dem Wunsch, einen kleineren Rohling zu verwenden, dann die Möglichkeit, diese Differenz der Dickenabmessungen durch Unterlegplatten zu kompensieren.

Eine besonders vorteilhafte Ausführungsform eines solchen Doppelwerkstückhalters veranschaulicht die modifizierte Querschnittsdarstellung der Abbildung 11 b. Die DickenAbmessungen der Rohlinge haben keinen Einfluss auf die in der A-Achse zentrierte Kontaktfläche (132, 133), da der obere Rohling (130) auf dem Plateau (142) der einen Grundplattenseite (140) und der untere Rohling (131) auf dem Plateau (143) der rechten Grundplattenseite (141) aufliegt. Beide Rohlinge liegen auf der gegenüberliegenden Seite ihrer Plateau-Auflage wiederum direkt auf dem gegenüberliegenden Rohling auf. Die Verbindung der beiden Grundplattenseiten (140, 141) findet über den umlaufenden Rahmen (144), der von den Klemmplatten (112) verdeckt wird, statt. Durch die Doppeldeutung der Skizze (s.o.) soll an dieser Stelle noch einmal für die hiesige Darstellung klargestellt werden, dass der Grundplattenanteil (145) zu beseitigenistunddassdie Kontaktflächen (132, 133) der Rohlinge (130, 131) bis zur A-Achslinie auszudehnen sind.
Ein sehr ähnliches Ausführungsbeispiel gemäß Anspruch 15, wie hier beschrieben, ist nochmals in den Abbildungen 9 j in Querschnittsdarstellung und 9 k in Frontansicht skizziert. Hier trägt der Rahmen (144) die Klemmschrauben (113), die direkt mit den Rohlingen in Kontakt stehen. Eine Blattfeder (170) gewährleistet den Kontakt zur gegenüberliegenden Wand (171) des Rahmens(144). Zusätzlich lassen sich die Rohlinge über eine abnehmbare Positionierungs-Vorrichtung (172) gemäß Anspruch 16 stufenweise verschieben.

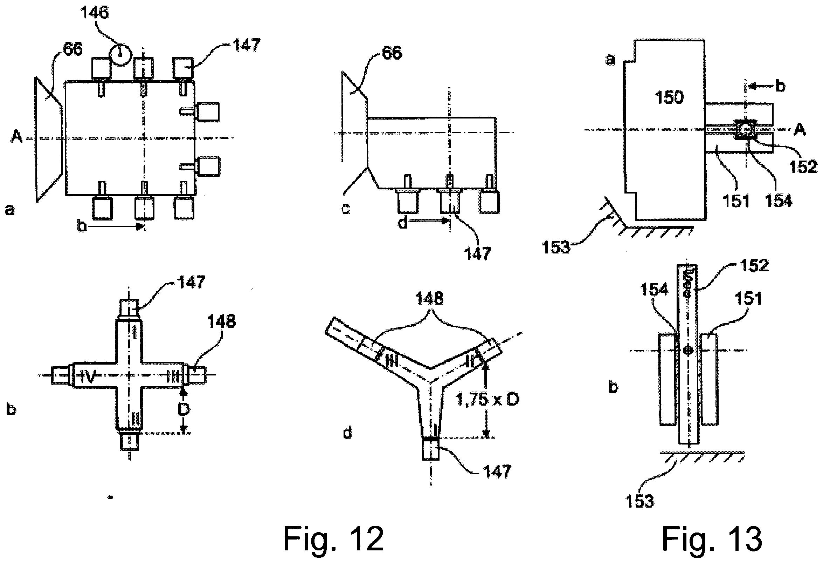

Die weit verbreiteten Einzelzahnrohlinge mit metallischen Befestigungsstiften für das CEREC- und CELAY- System lassen sich in bekannten Haltevorrichtungen mit Aufnahme von bis zu acht Einzelzahn-Rohlingen in vielen Maschinen in konventioneller Weise bearbeiten. Da die Rohlinge gemäß der Ableitung von Ronden-Haltern jeweils ins Zentrum des Werktisches weisen, sind sie einer stirnseitigen Bearbeitung nicht zugänglich. In Abbildung 12 sind zwei CEREC-Halter für alle erfindungsgemäßen Bearbeitungsstrategien dargestellt, die zudem die Bearbeitung von bis zu 14 Blöcken ermöglichen. Ihre Anzahl könnte noch weiter erhöht werden, indem die Abstände zwischen den einzelnen Blöcken von hier 18 mm auf bis zu 3,2 mm verkleinert werden.

Die Abbildungen 12a und c zeigen die Haltevorrichtung im Frontalquerschnitt in den Grundpositionen. Als Werkzeug (146) ist eine 18-mm-Walze mit H = 2 mm in der Grundposition (A0BO) skizziert. Drei Blöcke (147) sind jeweils in Reihe angeordnet. Die im Vorder- (III, II) und Hintergrund (IV, III) angeordneten Blöcke (148) sind nach entsprechender A-Achseinstellung gleichermaßen bearbeitbar. Die seitlichen vier Rohlinge (149) sind nur für Maschinenausstattungen, die die B = 90°-Position einnehmen können, vorgesehen. Um dem Bestreben möglichst viele Blöcke (Nachtbetrieb) unterzubringen, nachzukommen, ist es bei genannter Maschinenausstattung zweckmäßig, anstatt der hier gezeigten quadratischen oder rechteckigen Halter-Anordnung, die Rohlinge in Form eines Halb- oder 3/4-Kreises anzuordnen. Unter Berücksichtigung der weiteren beiden Schenkel (III, IV / II, III) würden die Rohlinge (147, 148, 149) dann auf einer Halb- bzw. 3/4-Kugel angeordnet sein. Hierbei wird durch die Nutzung der konvexen Anordnung die Auffächerung der Rohlinge Platzgewinn realisiert. Als vorteilhaft bei der dreischenkeligen Anordnung (7c, 7d) erweist sich der Platzgewinn für die Bewegungsfreiheit der Spindel, der sich gegenüber der kreuzförmigen (7a, b) Anordnung mit Faktor 1,75 auswirkt (D, 1,75 x D).

In Abbildung 13 ist ein hydraulischer, zentrierend spannender Schraubstock (150) mit auswechselbaren Klemmbacken (151) grob vereinfacht skizziert. Diese Ausführung ermöglicht ein einfaches und schnelles Befestigen der Rohlinge (152) gegebenenfalls mit Adapter (154) und lässt bei vielen Maschinenausführungen, limitiert durch die Maschinengeometrie (153), Längenabmessungen der Werkstücke von 140 mm zu.

Die in den Abbildungen 9e, 9f, 9g und 13 beschriebenen Ausführungen lassen sich natürlich ebenfalls mit Rohlingen des CEREC- oder CELAY-Systems bestücken. Ebenso können die in Abbildung 12 beschriebenen vorteilhaften Anordnungen auch mit Stangenmaterialien mittels direkter Klemmwirkung bestückt werden.

Im Besonderen soll abschließend noch einmal auf die Kombinationsmöglichkeit der in den Abbildungen 9, 12, 13 erläuterten Wirkungseffekte mit der der Kleberahmen-Bestückung gemäß den Erläuterungen zu Abbildung 6 eingegangen werden. Halterlose Rohlinge (E-max C14 Fa. Ivoclar, BZ 33 Fa. Vita) lassen sich nach Vorbehandlung (Sandstrahlen, Konditionierung, Haftvermittler) mit Miniaturausführungen der "L"-förmigen Rahmen verkleben, wobei deren Klemmvorrichtung an ihrer Befestigungsbasis mit einem CEREC-Stift ausgestattet sind.

Eine weitere Möglichkeit besteht darin, den metallischen Haltestift eines verbrauchten CEREC-Rohlings oder einen hierfür speziell angefertigten Befestigungsstift mit einem fräsbaren Rahmen zu verkleben, der an seiner gegenüberliegenden Seite wiederum mit einem halterlosen Rohling verklebt wird. Hierbei findet neben der vollflächigen Verklebung an seiner Stirnfläche (VI) zusätzlich eine sich nur über wenige Millimeter (1 mm bis 5 mm) erstreckende Verklebung an seinen angrenzenden Flächen I, II, IV V in Form einer sockelartigen Befestigung (Kerzenständer) oder an seinen angrenzenden, sich jeweils gegenüberliegenden Flächen I und II bzw. IV und V in Art einer U-förmigen Fassung statt. Auf diese Weise wird eine stabile Klebeverbindung bei geringer Kontaktfläche realisiert.

## Patentansprüche

1. Verfahren zur materialabtragenden Bearbeitung eines insbesondere flächigen Rohlings (41, 72, 73, 81, 134, 135, 152) zur Herstellung von dentalen Werkstücken (1, 130, 131), wie Inlays, Kronen, Brücken, Stegen, Implantatsuprakonstruktionen, Prothesen, Modellen oder dergleichen bei dem mit einem rotierenden Werkzeug (146) abwechselnd eine Kroneninnenseite des Werkstücks (1, 130, 131) in der Intrados-Position (57, 58) des Rohlings (41, 72, 73, 81, 134, 135, 152) und eine Kronenaußenseite in der Extrados-Position (60, 61) des Rohlings (41, 72, 73, 81, 134, 135, 152) bearbeitet wird, wobei das rotierende Werkzeug (146), insbesondere ein Fräswerkzeug, Bohrer oder Schleifwerkzeug sowohl stirnseitig als auch mantelseitig mit dem Werkstück (1, 130, 131) in Eingriff gebracht wird und wobei eine gleichmäßige Nutzung der gesamten Schneidfläche angestrebt ist und damit ein mantelseitiger Eingriff bevorzugt wird, **dadurch gekennzeichnet, dass** bei der Bearbeitung zusätzlich zu der Zustellung und dem Vorschub des rotierenden Werkzeugs (146) eine Werkzeugachsen bzw. Werkzeugmantelflächen parallele alternierende Auf- und Abbewegung als Huben stattfindet.

2. Verfahren nach Anspruch 1, **dadurch gekennzeichnet, dass** die Bearbeitung des Werkstücks (1, 130, 131), die wenigstens den Schrupp-Vorgang betrifft, zu einem Teil, gemäß eines horizontal formenden Verfahrens mit zunehmender oder abnehmender Z-Zustellung und zu einem anderen Teil, gemäß eines vertikal formenden Verfahrens stattfindet.

3. Verfahren nach zumindest einem der vorhergehenden Ansprüche, **dadurch gekennzeichnet, dass** die Bearbeitung des Rohlings (41, 72, 73, 81, 134, 135, 152) an einer freien Seite im Winkelbereich der Wendeachse von A = 0° bis 180° respektive A = 180° bis 360° stattfindet, oder A = (α-min) bis 0° bis 180° bis (α-max), respektive A = (α-min) bis 180° bis 360° bis (α-max) stattfindet, und/oder kombiniert mit der Einstellung der Schwenkachse (78) im Winkelbereich von B = 0° bis 90° und/oder B = (β-min) bis 0° bis 90° bis (β-max) stattfindet, wobei die Winkelangaben α-min, α-max, β-min und β-max die jeweiligen Maschinen und Werkstück, das heißt, Kollisionsbedingten Grenzwinkel darstellen, und wobei neben den Standart-Grundpositionen B = 0° + A = 0°/90°/180°/270° zwei weitere Grundposition B = 90° + A = 0°/360° und B = 90° - A = 90°/270° vorliegen.

4. Verfahren nach zumindest einem der vorhergehenden Ansprüche, **dadurch gekennzeichnet, dass** das Werkstück (1, 130, 131) wenigstens den Schruppvorgang betreffend abschnittsweise bearbeitet wird, wobei die Abschnitte durch vier Tangentenlinien begrenzt werden, deren Tangenten-Winkel 45° betragen, und die Abschnitte mit ihren eher vertikal zur Intrados- bzw. Extrados-Oberfläche orientierten Werkstückoberflächen mit einer Werkzeugachsen-Neigung zur Intrados- bzw. Extrados-Oberfläche von 45°bis 90°, vorzugsweise 90°, und die Abschnitte mit horizontal orientierten Werkstückoberflächen (132, 133) mit einer Werkzeugachsen-Neigung in einem Winkelbereich 0° bis 45°, vorzugsweise 30° bis 45°, bearbeitet werden.

5. Verfahren nach zumindest einem der vorhergehenden Ansprüche, **dadurch gekennzeichnet, dass** zumindest in der Schrupp-Bearbeitung in einem Schritt die Bearbeitung mit annähernd senkrecht zur Längsachse der Restauration stattfindet und in einem anderen vorzugsweise vorhergehenden Schritt die Bearbeitung in einem Winkelbereich zwischen 0° und 45°, ausgerichteter WerkzeugRotationsachse stattfindet, wobei die Realisierung der bevorzugten kleineren Winkel im Sinne einer konstruktionsbedingten Kollision eingeschränkt wird.

6. Verfahren nach zumindest einem der vorhergehenden Ansprüche, **dadurch gekennzeichnet, dass** eine tangentiale Bearbeitung, wenigstens die Schrupp-Bearbeitung betreffend, entlang der Tangenten-Linie mit Tangenten-Winkel zwischen 0°und 90° bzw. 90° und 180° in mehreren festgelegten Schritten und/ oder durch die CAM-Software individuell ermittelten Winkelwerten mit kontinuierlich simultaner Anpassung stattfindet.

7. Verfahren nach zumindest einem der vorhergehenden Ansprüche, **dadurch gekennzeichnet, dass** die Bearbeitung durch eine Kombination des Hubens mit einem horizontal formenden Verfahren mit zunehmender oder abnehmender Z-Zustellung und/oder vertikal formenden Verfahren stattfindet.

8. Verfahren nach zumindest einem der vorhergehenden Ansprüche, **dadurch gekennzeichnet, dass** an den frei zugänglichen Stirnseiten (III) in einem frühen Schritt mittels eines scheibenförmigen Werkzeugs (146) nach Art einer Schneidvorrichtung, insbesondere einer Kreissäge, ein Abschnitt des Rohlings (41, 72, 73, 81, 134, 135, 152) abgetrennt wird.

9. Verfahren nach zumindest einem der vorangegangenen Ansprüche, **dadurch gekennzeichnet, dass** der Werktisch aus einem "U"-förmigen Werktischrahmen und in diesen in unverwechselbarer Position reponierbaren Werktischeinsätzen (102) zusammengesetzt ist.

10. Verfahren nach zumindest einem der vorhergehenden Ansprüche, **dadurch gekennzeichnet, dass** das Werkstück (1, 130, 131) mit einem geklebten Rahmen (144) aus einem leicht zu bearbeitenden Material (49, 75), insbesondere Kunststoff oder Leichtmetall, für die Befestigung in einem Werksstückhalter per Klemmwirkung verbunden ist, wobei der Rahmen (144) unter Freilassung von mindestens zwei Stirnseiten (III) "L"- bzw. "C"-förmig gestaltet ist und der von dem Werkstückhalter freigelassene Rahmenanteil eine Mindestbreite von dem Durchmesser des zumindest für diese Bearbeitungsseite verwendeten größten Werkzeugs (146) aufweist.

11. Verfahren nach zumindest einem der vorhergehenden Ansprüche, **dadurch gekennzeichnet, dass** in einem ersten Bearbeitungsschritt das Werkstück (1, 130, 131) per Klemmwirkung (185) mit dem Werkstückmaterial verbunden ist, wobei mindestens zwei Stirnseiten (III) frei zugänglich sind und nach dessen zumindest fortgeschrittenen Verbrauch in einem zweiten Bearbeitungsschritt mit Aufhebung der primären Befestigung und Freilegung des primären Klemmbereichs (83) eine erneute Befestigung stattfindet, ohne dass dabei eine Positionsänderung zwischen Werkstück (1, 130, 131) und Werktisch eintreten kann.

12. Verfahren nach zumindest einem der vorangegangenen Ansprüche bei unilateraler Werktischbefestigung, **dadurch gekennzeichnet, dass** mindestens zwei Rohlinge (41, 72, 73, 81, 134, 135, 152) je nach Orientierung mit ihren Längs- oder Querseiten parallel zur Wendeachse über ihre gesamte Länge bzw. Breite durch Klemmwirkung (185) befestigt sind, die an ihren freien gegenüberliegenden Enden bearbeitet werden können, wobei ihre Quer- bzw. Längsachse jeweils die Wendeachse schneidet.

13. Verfahren nach zumindest einem der vorangegangenen Ansprüche bei unilateraler Werktischbefestigung, **dadurch gekennzeichnet, dass** mindestens zwei Rohlinge je nach Orientierung mit ihren Längs- oder Querseiten parallel zur Wendeachse über ihre gesamte Länge bzw. Breite durch Klemmwirkung (185) befestigt sind, die an ihren freien gegenüberliegenden Enden bearbeitet werden können, wobei die Rohlinge (41, 72, 73, 81, 134, 135, 152) beiderseits der Wendeachse angeordnet sind und nur im Fall ihres direkten Kontaktes miteinander die Wendeachse in ihrer Kontaktfläche (136) enthalten.

14. Verfahren nach zumindest einem der vorangegangenen Ansprüche, **dadurch gekennzeichnet, dass** nur ein Teil des Rohlings (41, 72, 73, 81, 134, 135, 152) von drei Stirnseiten (III) frei zugänglich ist, der Restteil in der Befestigungsvorrichtung fixiert ist und nach Verbrauch des freien Teils eine stufenweise Verschiebung des Rohlings (41, 72, 73, 81, 134, 135, 152) mittels Repositionshilfen stattfindet, wobei nach Berücksichtigung der Positionsänderung in der CAM-Software die weitere Bearbeitung des hiermit frei zugänglichen Teils stattfinden kann.

15. Verfahren nach zumindest einem der vorangegangenen Ansprüche zur Bearbeitung von mit Metallstift zu befestigenden (41, 72, 73, 81, 134, 135, 152), **dadurch gekennzeichnet, dass** bei
1. bilateraler Werktischbefestigung
1.1 zweimal ein bis mehrere Rohlinge (41, 72, 73, 81, 134, 135, 152) in 120° Anordnung,
1.2 dreimal ein bis mehrere Rohlinge (41, 72, 73, 81, 134, 135, 152) in 90° Anordnung beziehungsweise bei 2. unilateraler Werktischbefestigung
2.1 zweimal ein bis mehrere Rohlinge (41, 72, 73, 81, 134, 135, 152) in 180°-Anordnung,
2.2 dreimal ein bis mehrere Rohlinge (41, 72, 73, 81, 134, 135, 152) in120°-Anordnung,
2.3 viermal ein bis mehrere Rohlinge (41, 72, 73, 81, 134, 135, 152) in 90°-Anordnung,
arrangiert werden.

## Claims

1. Method for the material-removing machining of an in particular planar blank (41, 72, 73, 81, 134, 135, 152) for the production of dental workpieces (1, 130, 131), such as inlays, crowns, bridges, bars, implant superstructures, prostheses, models or the like, which method involves alternately machining a crown inner side of the workpiece (1, 130, 131) in the intrados position (57, 58) of the blank (41, 72, 73, 81, 134, 135, 152) and a crown outer side in the extrados position (60, 61) of the blank (41, 72, 73, 81, 134, 135, 152) using a rotating tool (146), wherein the rotating tool (146), in particular a milling tool, drill or grinding tool, is brought into engagement with the workpiece (1, 130, 131) both frontally and also circumferentially, and wherein uniform utilization of the entire cutting surface is sought and, therefore, a circumferential engagement is preferred, **characterized in that** in the machining, in addition to the infeed and advance of the rotating tool (146), an alternating upward and downward movement parallel to tool axes or tool circumference surfaces takes place in the form of excursions.

2. Method according to Claim 1, **characterized in that** the machining of the workpiece (1, 130, 131), which at least concerns the roughing process, is effected, on the one hand, in accordance with a horizontal shaping method, with an increasing or decreasing Z-infeed, and, on the other hand, in accordance with a vertical shaping method.

3. Method according to at least one of the preceding claims, **characterized in that** the machining of the blank (41, 72, 73, 81, 134, 135, 152) takes place on a free side in the angle range of the turning axis of A = 0° to 180° or A = 180° to 360°, or A = (α-min) to 0° to 180° to (α-max), or A = (α-min) to 180° to 360° to (α-max), and/or in combination with the adjustment of the pivot axis (78) in the angle range of B = 0° to 90° and/or B = (β-min) to 0° to 90° to (β-max), wherein the angle indications α-min, α-max, β-min and β-max represent the respective machines and workpiece, that is to say collision-related limit angles, and wherein, in addition to the standard basic positions B = 0° + A = 0°/90°/180°/270°, two further basic positions B = 90° + A = 0°/360° and B = 90° - A = 90°/270° are present.

4. Method according to at least one of the preceding claims, **characterized in that** the workpiece (1, 130, 131), at least as regards the roughing process, is machined in portions, wherein the portions are delimited by four tangent lines whose tangent angles are 45°, and the portions whose workpiece surfaces are oriented more vertically to the intrados or extrados surface are machined with a tool axis inclination to the intrados or extrados surface of 45° to 90°, preferably 90°, and the portions with horizontally oriented workpiece surfaces (132, 133) are machined with a tool axis inclination in an angle range of 0° to 45°, preferably 30° to 45°.

5. Method according to at least one of the preceding claims, **characterized in that**, at least in the rough machining, in one step the machining takes place approximately perpendicular to the longitudinal axis of the restoration and, in another step, preferably a preceding step, the machining takes place with a tool rotation axis oriented in an angle range of between 0° and 45°, wherein the realization of the preferred smaller angles is limited in the sense of a construction-related collision.

6. Method according to at least one of the preceding claims, **characterized in that** a tangential machining, at least as regards the rough machining, is effected along the tangent line with a tangent angle of between 0° and 90° or between 90° and 180° in several fixed steps and/or angle values individually determined by the CAM software with continuously simultaneous adaptation.

7. Method according to at least one of the preceding claims, **characterized in that** the machining is effected by a combination of the excursion with a horizontal shaping method with increasing or decreasing Z-infeed and/or a vertical shaping method.

8. Method according to at least one of the preceding claims, **characterized in that**, at the freely accessible front faces (III), a portion of the blank (41, 72, 73, 81, 134, 135, 152) is cut away in an early step by means of a disc-shaped tool (146) in the manner of a cutting device, in particular a circular saw.

9. Method according to at least one of the preceding claims, **characterized in that** the workbench is composed of a U-shaped workbench frame and of workbench inserts (102) that can be repositioned into the latter in a unique position.

10. Method according to at least one of the preceding claims, **characterized in that** the workpiece (1, 130, 131) is connected to a bonded frame (144) made of an easily machinable material (49, 75), in particular a plastic or a light metal, for fastening in a workpiece holder by a clamping action, wherein the frame (144) is L-shaped or C-shaped, leaving free at least two front faces (III), and the frame part left free by the workpiece holder has a minimum width corresponding to the diameter of the at least largest tool (146) used for this machining side.

11. Method according to at least one of the preceding claims, **characterized in that**, in a first machining step, the workpiece (1, 130, 131) is connected to the workpiece material by a clamping action (185), wherein at least two front faces (III) are freely accessible and, after the at least advanced consumption thereof, in a second machining step a renewed fastening takes place with cancellation of the primary fastening and freeing of the primary clamping area (83), without a change of position being able to occur between workpiece (1, 130, 131) and workbench.

12. Method according to at least one of the preceding claims with unilateral workbench fastening, **characterized in that** at least two blanks (41, 72, 73, 81, 134, 135, 152) are fastened, depending on the orientation, by a clamping action (185) along their entire length or width with their longitudinal sides or transverse sides parallel to the turning axis and can be machined at their free opposite ends, wherein their transverse or longitudinal axis in each case intersects the turning axis.

13. Method according to at least one of the preceding claims with unilateral workbench fastening, **characterized in that** at least two blanks are fastened, depending on the orientation, by a clamping action (185) along their entire length or width with their longitudinal sides or transverse sides parallel to the turning axis and can be machined at their free opposite ends, wherein the blanks (41, 72, 73, 81, 134, 135, 152) are arranged on both sides of the turning axis and contain the turning axis in their contact face (136) only in the case of their direct contact with each other.

14. Method according to at least one of the preceding claims, **characterized in that** only a part of the blank (41, 72, 73, 81, 134, 135, 152) is freely accessible from three front faces (III), the remaining part is fixed in the fastening device and, after consumption of the free part, a stepwise displacement of the blank (41, 72, 73, 81, 134, 135, 152) is effected by means of repositioning aids, wherein the further machining of the then freely accessible part can take place after the change of position has been taken into consideration in the CAM software.

15. Method according to at least one of the preceding claims for the machining of (41, 72, 73, 81, 134, 135, 152) to be fastened with a metal pin, **characterized in that**
1. with bilateral workbench fastening
1.1 one to several blanks (41, 72, 73, 81, 134, 135, 152) are arranged twice at 120°,
1.2 one to several blanks (41, 72, 73, 81, 134, 135, 152) are arranged three times at 90°, or
2. with unilateral workbench fastening
2.1 one to several blanks (41, 72, 73, 81, 134, 135, 152) are arranged twice at 180°,
2.2 one to several blanks (41, 72, 73, 81, 134, 135, 152) are arranged three times at 120°, or
2.3 one to several blanks (41, 72, 73, 81, 134, 135, 152) are arranged four times at 90°.

## Revendications

1. Procédé d'usinage par enlèvement de matière d'une ébauche (41, 72, 73, 81, 134, 135, 152) notamment plane en vue de fabriquer des pièces dentaires (1, 130, 131), telles que des prothèses intrinsèques, des couronnes, des bridges, des pivots, des supraconstructions d'implant, des prothèses, des modèles ou similaires, avec lequel un côté intérieur de couronne de la pièce (1, 130, 131) dans la position intrados (57, 58) de l'ébauche (41, 72, 73, 81, 134, 135, 152) et un côté extérieur de couronne dans la position extrados (60, 61) de l'ébauche (41, 72, 73, 81, 134, 135, 152) sont usinés en alternance avec un outil en rotation (146), l'outil en rotation (146), notamment un outil de fraisage, un foret ou un outil de rectification, étant amené en prise avec la pièce (1, 130, 131) à la fois du côté frontal et du côté de l'enveloppe et une utilisation homogène de la totalité de la surface de coupe étant visée et une prise côté enveloppe étant ainsi préférée, **caractérisé en ce que** lors de l'usinage, en plus de l'approche et de l'avance de l'outil en rotation (146), il se produit un mouvement montant et descendant alterné parallèle à des axes d'outil ou à des surfaces d'enveloppe d'outil sous la forme d'excursions.

2. Procédé selon la revendication 1, **caractérisé en ce que** l'usinage de la pièce (1, 130, 131), lequel concerne au moins l'opération de dégrossissage, a lieu d'une part selon un procédé de façonnage horizontal avec approche Z croissante ou décroissante, et d'autre part selon un procédé de façonnage vertical.

3. Procédé selon au moins l'une des revendications précédentes, **caractérisé en ce que** l'usinage de l'ébauche (41, 72, 73, 81, 134, 135, 152) a lieu sur un côté libre dans la plage angulaire de l'axe de retournement de A = 0° à 180° et de A = 180° à 360°, ou de A = (α-min) à 0° à 180° à (α-max), ou de A = (α-min) à 180° à 360° à (α-max), et/ou a lieu en combinaison avec le réglage de l'axe de pivotement (78) dans la plage angulaire de B = 0° à 90° et/ou B = (β-min) à 0° à 90° à (β-max), les indications angulaires α-min, α-max, β-min et β-max représentant respectivement les angles limites de machine et de pièce, c'est-à-dire conditionnés par la collision, et deux autres positions de base B = 90° + A = 0°/360° et B = 90° - A = 90°/270° étant présentes en plus des positions de base standard B = 0° + A = 0°/90°/180°/270°.

4. Procédé selon au moins l'une des revendications précédentes, **caractérisé en ce que** la pièce (1, 130, 131), au moins pour ce qui concerne l'opération de dégrossissage, est usinée par portions, les portions étant délimitées par quatre lignes de tangente dont les angles de tangente sont de 45°, et les portions dont les surfaces de pièce sont orientées plutôt verticalement par rapport à la surface d'intrados ou d'extrados sont usinées avec une inclinaison de l'axe d'outil par rapport à la surface d'intrados ou d'extrados de 45° à 90°, de préférence de 90°, et les portions dont les surfaces de pièce (132, 133) sont orientées horizontalement avec une inclinaison de l'axe d'outil dans une plage angulaire de 0° à 45°, de préférence de 30° à 45°.

5. Procédé selon au moins l'une des revendications précédentes, **caractérisé en ce qu'**au moins lors de l'usinage de dégrossissage, l'usinage est effectué dans une étape approximativement perpendiculairement à l'axe longitudinal de la restauration et, dans une autre étape de préférence précédente, l'usinage est effectué avec un axe de rotation d'outil orienté dans une plage angulaire entre 0° et 45°, la réalisation des angles de préférence plus petits dans le sens d'une collision liée à la construction étant limitée.

6. Procédé selon au moins l'une des revendications précédentes, **caractérisé en ce qu'**un usinage tangentiel, concernant au moins l'usinage de dégrossissage, a lieu le long de la ligne tangentielle avec un angle de tangente entre 0° et 90° ou entre 90° et 180° au cours de plusieurs étapes définies et/ou des valeurs d'angle déterminées individuelles par le logiciel de FAO avec adaptation simultanée continuelle.

7. Procédé selon au moins l'une des revendications précédentes, **caractérisé en ce que** l'usinage est réalisé par une combinaison de l'excursion avec un procédé de façonnage horizontal avec approche Z croissante ou décroissante et/ou un procédé de façonnage vertical.

8. Procédé selon au moins l'une des revendications précédentes, **caractérisé en ce qu'**une portion de l'ébauche (41, 72, 73, 81, 134, 135, 152) est sectionnée au niveau des côtés frontaux (III) librement accessibles au cours d'une étape précédente au moyen d'un outil en forme de disque (146) de type dispositif de coupe, notamment une scie circulaire.

9. Procédé selon au moins l'une des revendications précédentes, **caractérisé en ce que** la table de travail est composée d'un cadre de table de travail en forme de « U » et d'inserts de table de travail (102) repositionnables dans celui-ci dans une position unique.

10. Procédé selon au moins l'une des revendications précédentes, **caractérisé en ce que** la pièce (1, 130, 131) est reliée avec un cadre collé (144) en un matériau (49, 75) facile à usiner, notamment une matière plastique ou un métal léger, servant à la fixation dans un porte-pièce par effet de serrage, le cadre (144) étant réalisé en forme de « L » ou de « C » en laissant au moins deux côtés frontaux (III) libres et la partie du cadre qui est laissée libre par le porte-pièce présente une largeur minimale correspondant au diamètre du plus grand outil (146) au moins utilisé pour ce côté de l'usinage.

11. Procédé selon au moins l'une des revendications précédentes, **caractérisé en ce que** dans une première étape d'usinage, la pièce (1, 130, 131) est reliée au matériau de pièce par effet de serrage (185), au moins deux côtés frontaux (III) étant librement accessibles et après leur consommation au moins avancée, dans une deuxième étape d'usinage, une nouvelle fixation a lieu avec annulation de la fixation primaire et libération de la zone de serrage primaire (83) sans qu'un changement de position ne puisse ici se produire entre la pièce (1, 130, 131) et la table de travail.

12. Procédé selon au moins l'une des revendications précédentes lors d'une fixation unilatérale sur table de travail, **caractérisé en ce qu'**au moins deux ébauches (41, 72, 73, 81, 134, 135, 152), suivant l'orientation, sont fixées par effet de serrage (185) sur toute leur longueur ou largeur, avec leurs côtés longitudinaux ou transversaux parallèles à l'axe de retournement, lesquelles peuvent être usinées au niveau de leurs extrémités opposées libres, leur axe transversal ou longitudinal croisant respectivement l'axe de retournement.

13. Procédé selon au moins l'une des revendications précédentes lors d'une fixation unilatérale sur table de travail, **caractérisé en ce qu'**au moins deux ébauches, suivant l'orientation, sont fixées par effet de serrage (185) sur toute leur longueur ou largeur, avec leurs côtés longitudinaux ou transversaux parallèles à l'axe de retournement, lesquelles peuvent être usinées au niveau de leurs extrémités opposées libres, les ébauches (41, 72, 73, 81, 134, 135, 152) étant disposées des deux côtés de l'axe de retournement et ne contenant l'axe de retournement dans leur surface de contact (136) que dans le cas de leur contact direct l'une avec l'autre.

14. Procédé selon au moins l'une des revendications précédentes, **caractérisé en ce qu'**une partie seulement de l'ébauche (41, 72, 73, 81, 134, 135, 152) est accessible librement depuis trois côtés frontaux (III), la partie restante est fixée dans le dispositif de fixation et, après consommation de la partie libre, un décalage par paliers de l'ébauche (41, 72, 73, 81, 134, 135, 152) est effectué au moyen d'aides au repositionnement, la poursuite de l'usinage de la partie ainsi devenue accessible pouvant être effectué après prise en compte du changement de position dans le logiciel de FAO.

15. Procédé selon au moins l'une des revendications précédentes pour l'usinage de (41, 72, 73, 81, 134, 135, 152) à fixer avec une tige métallique, **caractérisé en ce que**
1. lors d'une fixation bilatérale sur table de travail
1.1 deux fois une à plusieurs ébauches (41, 72, 73, 81, 134, 135, 152) sont disposées selon un arrangement à 120°,
1.2 trois fois une à plusieurs ébauches (41, 72, 73, 81, 134, 135, 152) sont disposées selon un arrangement à 90°, ou 2. lors d'une fixation unilatérale sur table de travail
2.1 deux fois une à plusieurs ébauches (41, 72, 73, 81, 134, 135, 152) sont disposées selon un arrangement à 180°,
2.2 trois fois une à plusieurs ébauches (41, 72, 73, 81, 134, 135, 152) sont disposées selon un arrangement à 120°,
2.3 quatre fois une à plusieurs ébauches (41, 72, 73, 81, 134, 135, 152) sont disposées selon un arrangement à 90°.
